# EUROPEAN PATENT SPECIFICATION

(11) **EP 3 836 847 B1**
(45) Date of publication and mention of the grant of the patent: **17.07.2024**
(21) Application number: 19850437.5
(22) Date of filing: 05.08.2019
(51) Int. Cl.: A61B 17/115, A61B 17/00, A61B 50/00, A61B 17/34, A61B 17/29, A61B 90/70, A61B 90/00

(54) **SURGICAL DEVICES INCLUDING FEATURES TO FACILITATE CLEANING**
CHIRURGISCHE VORRICHTUNGEN MIT MERKMALEN ZUR ERLEICHTERUNG DER REINIGUNG
DISPOSITIFS CHIRURGICAUX COMPRENANT DES ÉLÉMENTS POUR FACILITER LE NETTOYAGE

(30) Priority: 14.08.2018 US 201862718450 P; 14.08.2018 US 201862718445 P; 14.08.2018 US 201862718438 P
(43) Date of publication of application: 23.06.2021
(73) Proprietor: Covidien LP, Mansfield, MA 02048 (US)
(72) Inventor: WILLIAMS, Justin, Southbury, Connecticut 06488 (US); EISINGER, Joseph, Northford, Connecticut 06472 (US); PAUL, Stephen, Hartford, Connecticut 06118 (US); MOZDZIERZ, Patrick, Glastonbury, Connecticut 06033 (US); SGROI, Anthony, Wallingford, Connecticut 06492 (US); VALENTINE, David, Hamden, Connecticut 06518 (US); CABRERA, Ramiro, Cheshire, Connecticut 06410 (US); WINK, Jon, Haddam, Connecticut 06438 (US); RICHARD, Paul, Shelton, Connecticut 06484 (US); SAPIENZA, Jonathan, Orange, Connecticut 06477 (US)
(74) Representative: Maschio & Soames IP Ltd
(86) International application number: PCT/US2019/045051
(87) International publication number: WO 2020/036758

(56) References cited:
- EP-A2- 0 139 337
- WO-A1-2016/022919
- WO-A1-2018/140324
- WO-A1-99/58185
- CN-U- 203 059 954
- US-A1- 2010 094 260
- US-A1- 2014 190 523
- US-A1- 2016 296 234
- US-A1- 2017 095 269
- US-A1- 2017 172 700
- US-A1- 2017 181 606

## Description

### CROSS REFERENCE TO RELATED APPLICATIONS

The present application claims the benefit of and priority to U.S. Provisional Patent Application Serial No. 62/718,450, filed on August 14, 2018; U.S. Provisional Patent Application Serial No. 62/718,445, filed on August 14, 2018; and U.S. Provisional Patent Application Serial No. 62/718,438, filed on August 14, 2018.

### BACKGROUND

### Technical Field

The present disclosure relates generally to surgical devices. More specifically, the present disclosure relates to nozzles and/or plugs for use with reusable surgical devices which facilitate thoroughly cleaning the surgical devices.

### Background of Related Art

Surgical instruments including powered devices for use in surgical procedures are known. To permit reuse of the handle assemblies of these surgical instruments and so that the handle assembly may be used with a variety of end effectors, adapter assemblies and extension assemblies have been developed for selective attachment to the handle assemblies and to a variety of end effectors. Additionally, following use, the adapter, end effector and/or extension assemblies may be thoroughly cleaned and/or sterilized for reuse. PCT Application WO 2018/140324 describes a surgical stapler device with handle portion, and elongate stapler extending therefrom and a stapler head at distal end of the stapler shaft, the handle portion and stapler shaft having an internal channel extending up to the stapler head and the handle portion having gas inlet port extending transversely into the handle portion and intersecting with the internal channel. US patent application US 2010/094260 describes an adapter for a medical receptacle having a skirt with an outside diameter and a threaded inside diameter, and a tapered post within the skirt, the adapter includes a proximal end portion having a threaded surface including at least one thread configured to threadingly engage the threaded inside diameter of the skirt, a distal end portion having a surface portion and a cylindrical recess, a conduit positioned between the tapered recess and the cylindrical recess such that the conduit is in fluid communication with both the tapered recess and the cylindrical recess; and a longitudinal axis.

### SUMMARY

In one aspect according to the present invention there is provided a surgical device comprising:
an outer sleeve including an inner wall, a port and a housing within the port, the port extending through the inner wall of the outer sleeve; and
a luer configured for selective engagement with the housing, the luer including an input portion and an exit port, the input portion is disposed radially outward of the outer sleeve when the luer is engaged with the housing and is configured for engagement with a source of fluid, the exit port is disposed radially inward of the inner wall of the outer sleeve when the luer is engaged with the housing characterized in that the housing defines an aperture configured to direct fluid proximally.

The luer may include a threaded portion configured to selectively engage a tapped portion of the housing.

The surgical device may further comprise an end effector disposed distally of the outer sleeve and configured to treat tissue.

The surgical device may further comprise a seal within the outer sleeve and disposed proximally of the end effector.

The port may be disposed distally of the seal.

### BRIEF DESCRIPTION OF THE DRAWINGS

Embodiments of the present disclosure are described herein with reference to the figures 70 to 73 : Figures 1 to 69 and figures 74 to 78 do not relate to aspects of the invention.
FIG. 1 is a perspective separated view of an adapter assembly, , an extension assembly, , and an exemplary handheld electromechanical surgical device;
FIG. 2 is a perspective side view of the exemplary handheld electromechanical surgical device of FIG. 1;
FIG. 3 is a perspective side view of the adapter assembly of FIG. 1;
FIG. 4 is a perspective side view of the adapter assembly of FIG. 3 with the outer sleeve removed;
FIG. 5 is a perspective side view of the adapter assembly of FIGS. 3 and 4 with proximal and distal housings of first and second pusher assemblies removed;
FIG. 6 is a cross-sectional side view of the adapter assembly of FIGS. 2-4 taken along line 6-6 in FIG. 3;
FIG. 7 is a cross-sectional side view of the adapter assembly of FIGS. 2-5 taken along line 7-7 in FIG. 5;
FIG. 8 is an enlarged, perspective view of a coupling assembly and a transfer assembly of the adapter assembly of FIGS. 2-7;
FIG. 9 is a perspective side view of the adapter assembly of FIGS. 2-7 with the housing assemblies removed;
FIG. 10 is an enlarged view of the indicated area of detail of FIG. 9;
FIG. 11 is an enlarged view of the indicated area of detail of FIG. 6;
FIG. 12 is an enlarged view of the indicated area of detail of FIG. 7;
FIG. 13 is a perspective end view of the transfer assembly of FIG. 8;
FIG. 14 is an enlarged view of the indicated area of detail of FIG. 6;
FIG. 15 is an enlarged view of the indicated area of detail of FIG. 7;
FIG. 16 is an enlarged view of the indicated area of detail of FIG. 9;
FIG. 17 is a perspective side view of the extension assembly of FIG. 1;
FIG. 18 is a perspective side view of an inner flexible band assembly of the extension assembly of FIG. 17;
FIG. 19 is a perspective side view of an outer flexible band assembly of the extension assembly of FIG. 17;
FIG. 20 is a perspective side view of the inner and outer flexible band assemblies of FIGS. 18 and 19 and an exploded view of a frame assembly of the extension assembly of FIG. 17;
FIG. 21 is a perspective side view of the inner and outer flexible band assemblies and the frame assembly of FIG. 20;
FIG. 22 is an enlarged view of the indicated area of detail of FIG. 21;
FIG. 23 is a front, perspective view of the inner and outer flexible band assemblies and the frame assembly of FIG. 20;
FIG. 24 is an enlarged view of the indicated area of detail of FIG. 23;
FIG. 25 is a cross-sectional end view taken along line 25-25 of FIG. 17;
FIG. 26 is a cross-sectional end view taken along line 26-26 of FIG. 17;
FIG. 27 is an enlarged perspective side view of a distal end of the inner and outer flexible band assemblies and the frame assembly of FIG. 20 including a proximal seal member and first and second distal seal members;
FIG. 28 is an exploded perspective view of the proximal seal member and first and second distal seal members of FIG. 27;
FIG. 29 is an exploded view of a trocar assembly of the extension assembly of FIG. 17;
FIG. 30 is a perspective side view of the trocar assembly of FIG. 29;
FIG. 31 is a cross-sectional side view taken along line 31-31 of FIG. 30;
FIG. 32 is a cross-sectional top view taken along line 32-32 of FIG. 17;
FIG. 33 is an enlarge cross-sectional view of the distal end of the extension assembly of FIG. 17;
FIG. 34 is a perspective side view of the adapter assembly of FIG. 3 connected to the extension assembly of FIG. 17 and an end effector and an anvil assembly connected to the extension assembly;
FIG. 35 is an enlarged cross-sectional side view of the indicated area of detail of FIG. 34;
FIG. 36 is a rear, perspective view of an adapter assembly according to another embodiment of the present disclosure;
FIG. 37 is a perspective side view of the adapter assembly of FIG. 36 with an outer sleeve and a handle member removed;
FIG. 38 is a perspective side view of the adapter assembly of FIG. 37 with a base and a housing member removed;
FIG. 39 is a perspective side view of the adapter assembly of FIG. 38 with a support structure removed;
FIG. 40 is a cross-sectional side view taken along line 40-40 of FIG. 36;
FIG. 41 is a cross-sectional side view taken along line 41-41 of FIG. 40;
FIG. 42 is a rear, perspective view of an adapter assembly according to yet another embodiment of the present disclosure;
FIG. 43 is a cross-sectional side view taken along line 43-43 of FIG. 42;
FIG. 44 is a cross-sectional side view taken along line 44-44 of FIG. 42;
FIG. 45 is a perspective view of a connector assembly
FIG. 46 is an exploded perspective view of the connector assembly of FIG. 45;
FIG. 47 is a perspective view of the connector assembly of FIG. 45 with a sleeve and first section of a tubular extension removed;
FIG. 48 is a perspective view of the connector assembly of FIG. 45 with the sleeve removed;
FIG. 49 is a cross-sectional side view taken along line 49-49 of FIG. 45;
FIG. 50 is a perspective view, with parts separated, of a distal end of the adapter assembly of FIG. 1 in accordance with embodiments of the present disclosure;
FIG. 51 is a transverse cross-sectional view of a portion of the distal end of the adapter assembly of FIG. 50;
FIG. 52 is a longitudinal cross-sectional view of the distal end of the adapter assembly taken along line 52-52 of FIG. 50;
FIGS. 53 and 54 are perspective views of a distal portion of the adapter assembly of FIG. 50, with some parts removed;
FIG. 55 is a perspective view of a sensor assembly of the adapter assembly of FIG. 50;
FIG. 56 is a perspective view of a seal assembly for use with the frame assembly of FIG. 20;
FIG. 57 is an assembly view of the seal assembly of FIG. 56;
FIG. 58 is a perspective view of the seal assembly of FIGS. 56 and 57 shown within the frame assembly of FIG. 20;
FIG. 59 is a perspective view of the seal assembly of FIGS. 56 and 57 shown within the frame assembly of FIG. 20 and with portions of the frame assembly omitted;
FIG. 60 is a transverse cross-sectional view taken along line 60-60 of FIG. 58;
FIG. 61 is a longitudinal cross-sectional view taken along line 61-61 of FIG. 58;
FIG. 62 is an enlarged view of the indicated area of detail of FIG. 61;
FIG. 63 is a perspective view of a portion of a frame assembly in accordance with an embodiment of the present disclosure including a flushing port and a first type of direction nozzle;
FIG. 64 is an enlarged view of a portion of the frame assembly of FIG. 63;
FIG. 65 is a longitudinal cross-sectional view of a portion of the frame assembly of FIGS. 63 and 64;
FIG. 66 is a perspective view of a portion of the frame assembly of FIG. 63 including a second type of direction nozzle;
FIG. 67 is a longitudinal cross-sectional view of a portion of the frame assembly of FIGS. 66;
FIG. 68 is a perspective view of the second type of direction nozzle of FIGS. 66 and 67;
FIG. 69 is a perspective view of a portion of the frame assembly of FIG. 63 including a plug disposed therein;
FIG. 70 is a perspective view of a portion of a frame assembly in accordance with an embodiment of the present disclosure including a luer engaged therewith;
FIGS. 71 and 72 are cut-away views of a portion of the frame assembly and luer of FIG. 70;
FIG. 73 is an assembly view of a portion of the frame assembly and luer of FIGS. 70-72;
FIG. 74 is a perspective view of a portion of a frame assembly including an impeller engaged therewith;
FIGS. 75 and 76 are perspective views of the impeller of FIG. 74;
FIG. 77 is a cut-away view of a portion of the frame assembly and impeller of FIGS. 74-76; and
FIG. 78 is a schematic illustration of a robotic surgical system configured for use in accordance with the present disclosure.

### DETAILED DESCRIPTION OF EMBODIMENTS

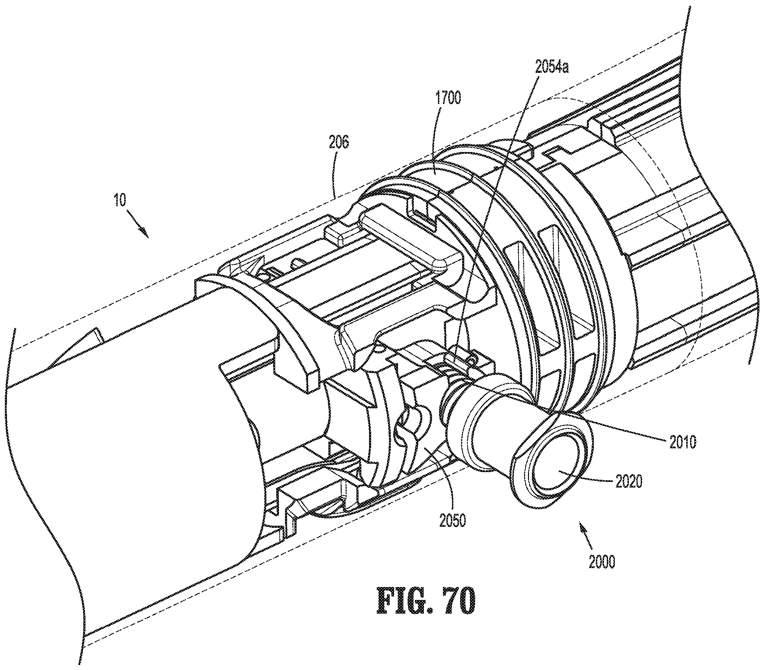

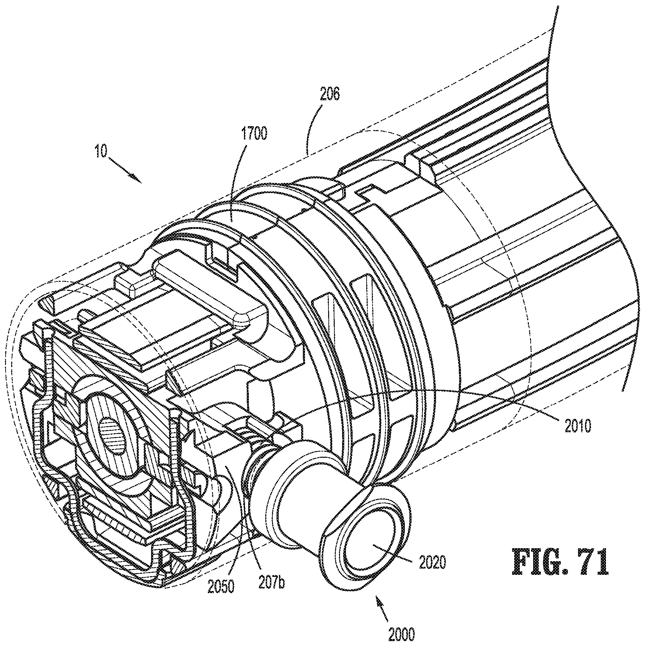

Embodiments of the presently disclosed seal assemblies for surgical instruments are described in detail with reference to the drawings of figures 70 to 73, in which like reference numerals designate identical or corresponding elements in each of the several views. As used herein the term "distal" refers to that portion of the seal assembly or surgical instrument, or component thereof, farther from the user, while the term "proximal" refers to that portion of the seal assembly or surgical instrument, or component thereof, closer to the user.

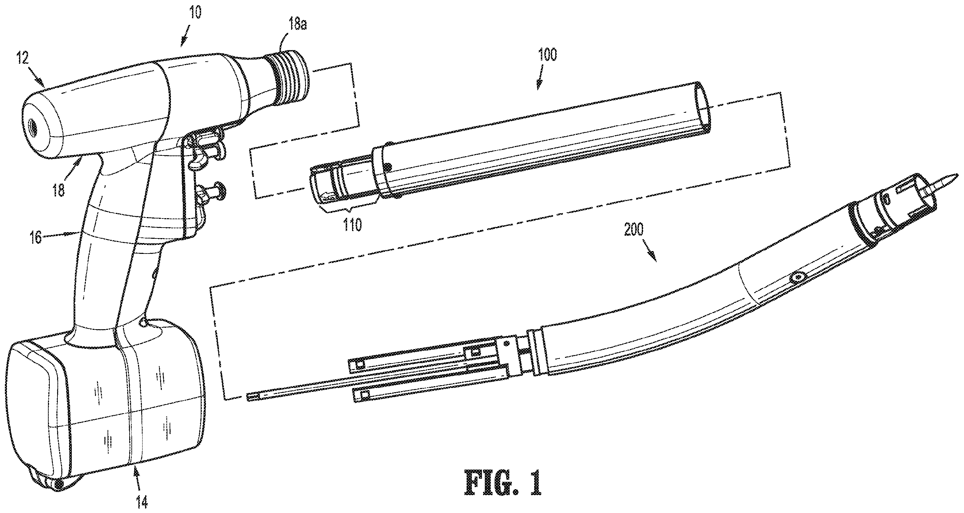

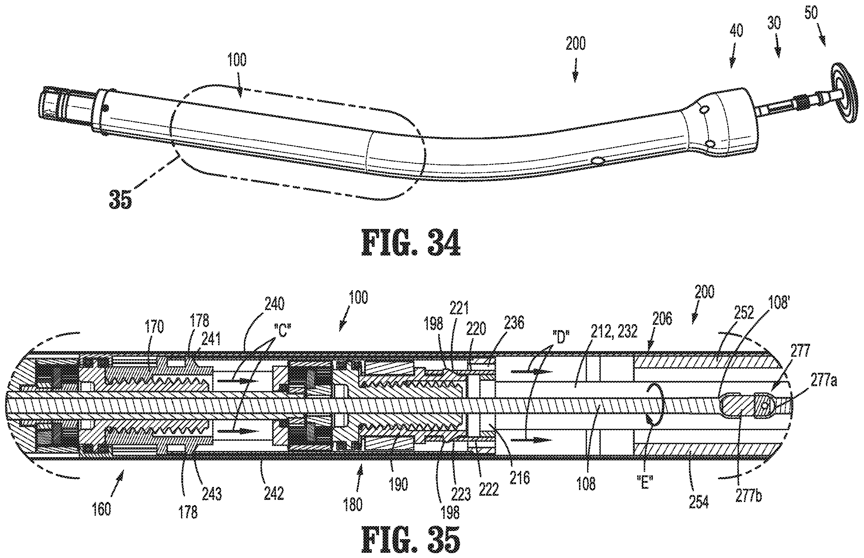

With reference to FIG. 1, an adapter assembly shown generally as adapter assembly 100, and an extension assembly, shown generally as extension assembly 200, are configured for selective connection to a powered handheld electromechanical instrument shown, generally as surgical device 10. As illustrated in FIG. 1, surgical device 10 is configured for selective connection with adapter assembly 100, and, in turn, adapter assembly 100 is configured for selective connection with an extension assembly 200. Extension assembly 200 is configured for selective connection with a tool assembly or end effector, e.g. tool assembly 30 (FIG. 34), including a loading unit, e.g. loading unit 40 (FIG. 34), and an anvil assembly, e.g., anvil assembly 50 (FIG. 34), for applying a circular array of staples (not shown) to tissue (not shown).

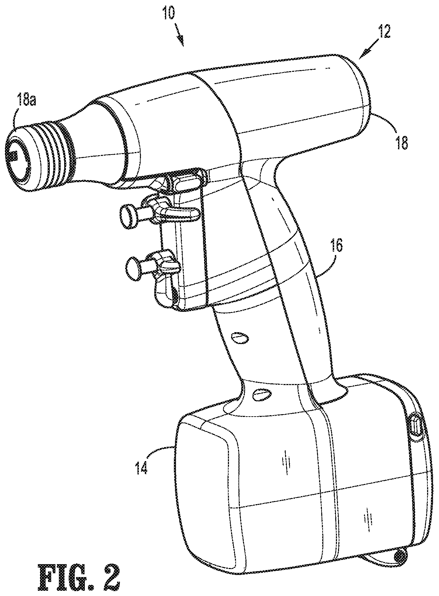

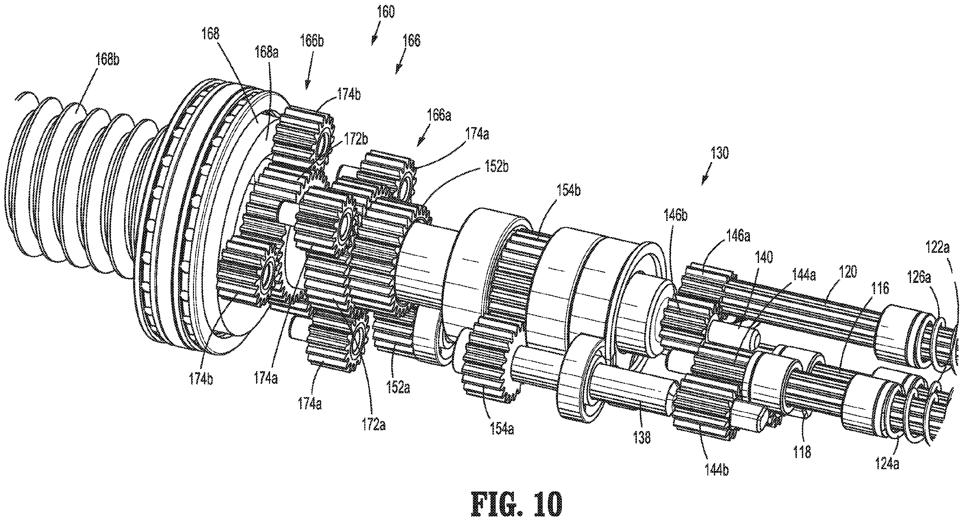

As illustrated in FIGS. 1 and 2, surgical device 10 includes a handle housing 12 having a lower housing portion 14, an intermediate housing portion 16 extending from and/or supported on lower housing portion 14, and an upper housing portion 18 extending from and/or supported on intermediate housing portion 16. A distal half-section of upper housing portion 18 defines a nose or connecting portion 18a configured to accept a corresponding drive coupling assembly 110 (FIG. 10) of adapter assembly 100. For a detailed description of the structure and function of an exemplary electromechanical instrument, please refer to commonly owned U.S. Patent 9,055,943 ("the '943 Patent"),.

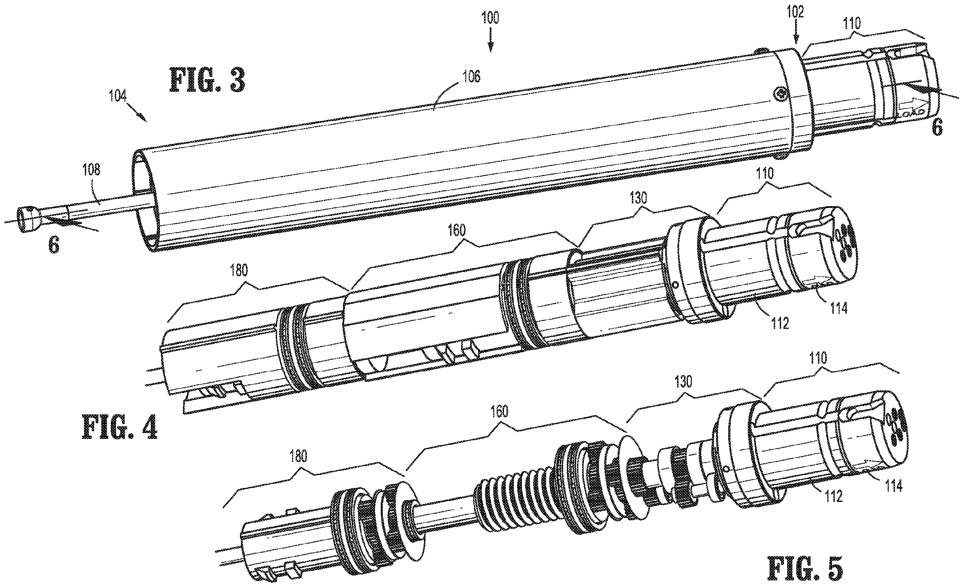

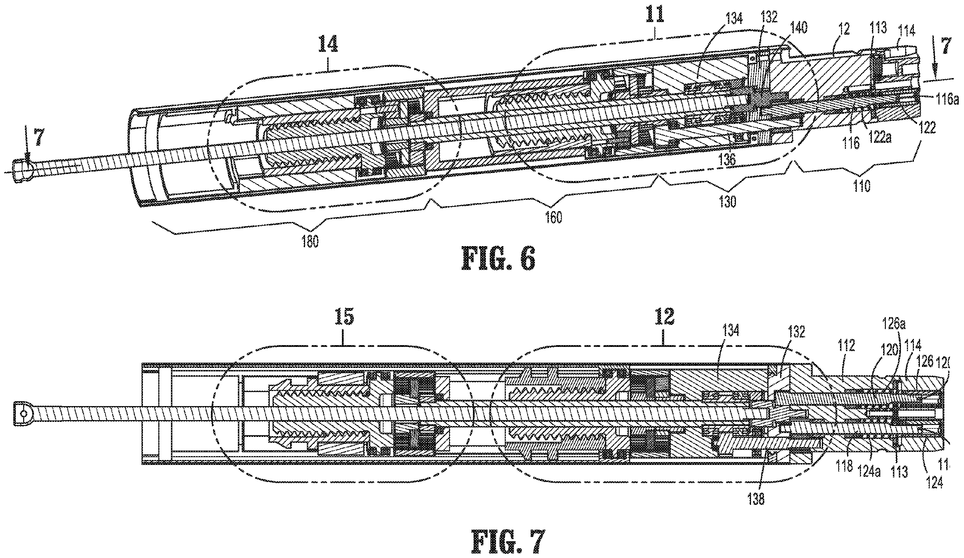

Adapter assembly 100 will now be described with reference to FIGS. 3-20. Referring initially to FIG. 3, adapter assembly 100 includes a proximal end 102 configured for operable connection to connecting portion 18a (FIG. 1) of surgical device 10 (FIG. 1) and a distal end 104 configured for operable connection to extension assembly 200 (FIG. 1). In accordance with the present disclosure, adapter assembly 100 may be substantially or fully rigid along the entire length.

Turning to FIGS. 3-5, from proximal end 102 to distal end 104 of adapter assembly 100, adapter assembly 100 includes a drive coupling assembly 110, a drive transfer assembly 130 operably connected to drive coupling assembly 110, a first pusher assembly 160 operably connected to drive transfer assembly 130, and a second pusher assembly 180 operably connected to drive transfer assembly 130. Each of drive transfer assembly 130, first pusher assembly 160 and second pusher assembly 180 are operably maintained within an outer sleeve 106 (FIG. 3). As will be described in further detail below, a shaft 108 (FIG. 3) extends longitudinally through adapter assembly 100 and is operably connected to drive transfer assembly 130.

With reference to FIGS. 5-9, drive coupling assembly 110 has a cylindrical profile and is configured to selectively secure adapter assembly 100 to surgical device 10 (FIG. 1). Drive coupling assembly 110 includes a connector housing 112 and a connector extension 114 fixedly connected to connector housing 112 by a mounting plate 113. Connector housing 112 and connector extension 114 operate to rotatably support a first rotatable proximal drive shaft 116, a second rotatable proximal drive shaft 118, and a third rotatable proximal drive shaft 120. Connector housing 112 and connector extension 114 of drive coupling assembly 110 also rotatably supports first, second, and third connector sleeves 116, 118, and 120, respectively. Each of connector sleeves 122, 124, 126 is configured to mate with respective first, second, and third drive connectors (not shown) of surgical device 10 (FIG. 1). Each connector sleeve 122, 124, 126 is further configured to mate with a proximal end 116a, 118a, 120a of respective first, second and third proximal drive shafts 116, 118, 120.

Drive coupling assembly 110 also includes first, second and third biasing members 122a, 124a and 126a disposed distally of respective first, second and third connector sleeves 122, 124, 126. Each of biasing members 122a, 124a and 126a is disposed about respective first, second, and third rotatable proximal drive shafts 122, 124 and 126 to help maintain connector sleeves 122, 124, and 126 engaged with the distal end of respective drive rotatable drive connectors (not shown) of surgical device 10 when adapter assembly 100 is connect to surgical device 10. In particular, first, second and third biasing members 122a, 124a and 126a function to bias respective connector sleeves 122, 124 and 126 in a proximal direction.

For a detailed description of an exemplary drive coupling assembly, please refer to the `943 Patent.

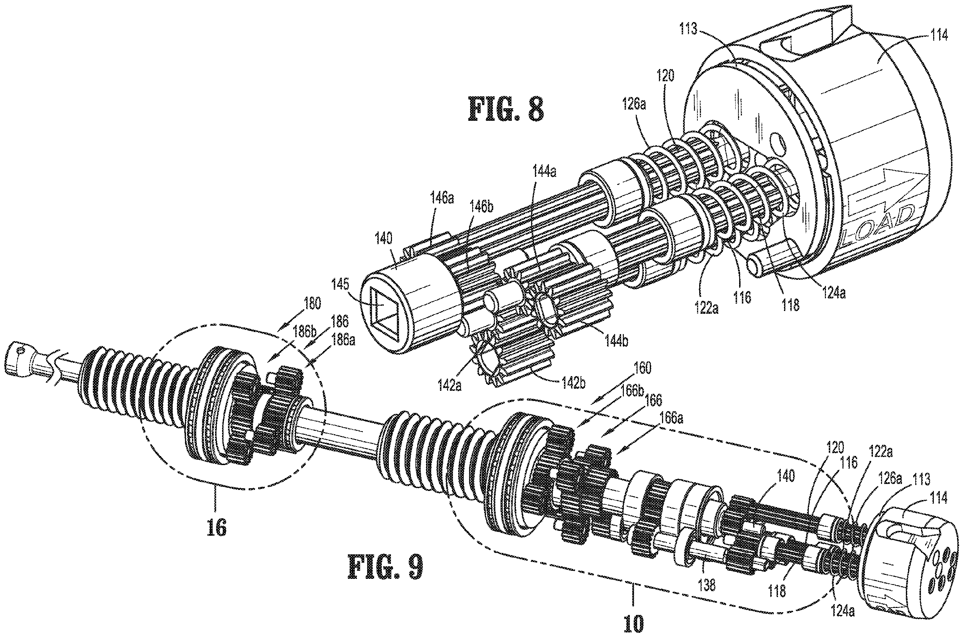

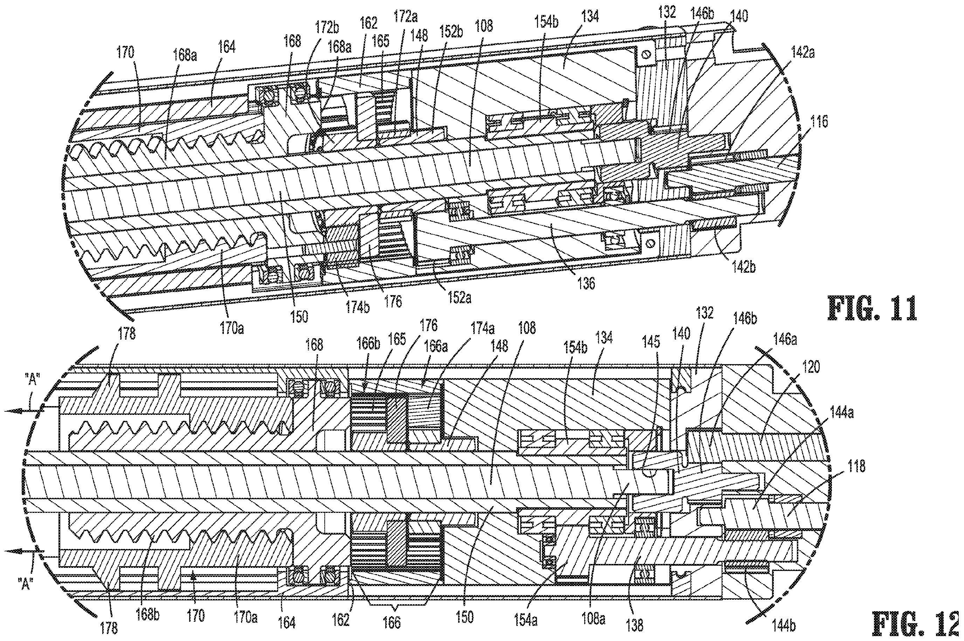

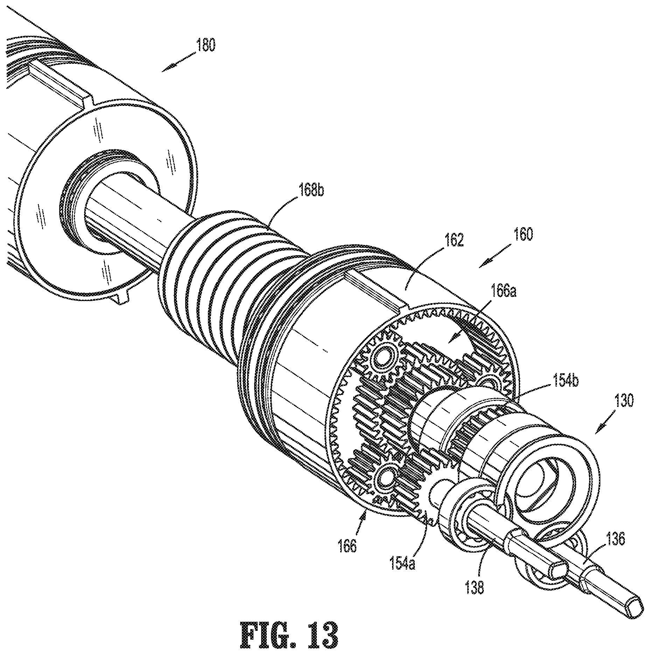

With reference to FIGS. 9-13, drive transfer assembly 130 (FIGS. 10 and 13) of adapter assembly 100 has a cylindrical profile and operably connects distal ends of first, second and third rotatable proximal drive shafts 116, 118 and 120 to shaft 108, first pusher assembly 160, and second pusher assembly 180, respectively. Drive transfer assembly 130 includes a support plate 132 (FIGS. 11 and 12) secured to a proximal end of connector housing 112 and a drive transfer housing 134 positioned adjacent support plate 132. Support plate 132 and housing 134 operate to rotatably support a first rotatable distal drive shaft 136, a second rotatable distal drive shaft 138 and a drive member 140.

First and second rotatable distal drive shafts 136 and 138 are each operably connected to respective first and second rotatable proximal drive shafts 116 and 118 of drive coupling assembly 110 by a pair of gears. In particular, distal ends of each of first and second rotatable proximal drive shaft 116 and 118 include a geared portion 142a and 144a, respectively, which engages a proximal drive gear 142b and 144b on a proximal end of respective first and second distal drive shafts 136 and 138. As shown, each of respective paired geared portion and proximal drive gear 142a, 142b and 144a, 144b are the same size to provide a 1:1 gear ratio between the respective rotatable proximal and distal drive shafts. In this manner, respective rotatable proximal and distal drive shafts rotate at the same speed. However, it is envisioned that either or both of the paired geared portions and proximal drive gears may be of different sizes to alter the gear ratio between the rotatable proximal and distal drive shafts.

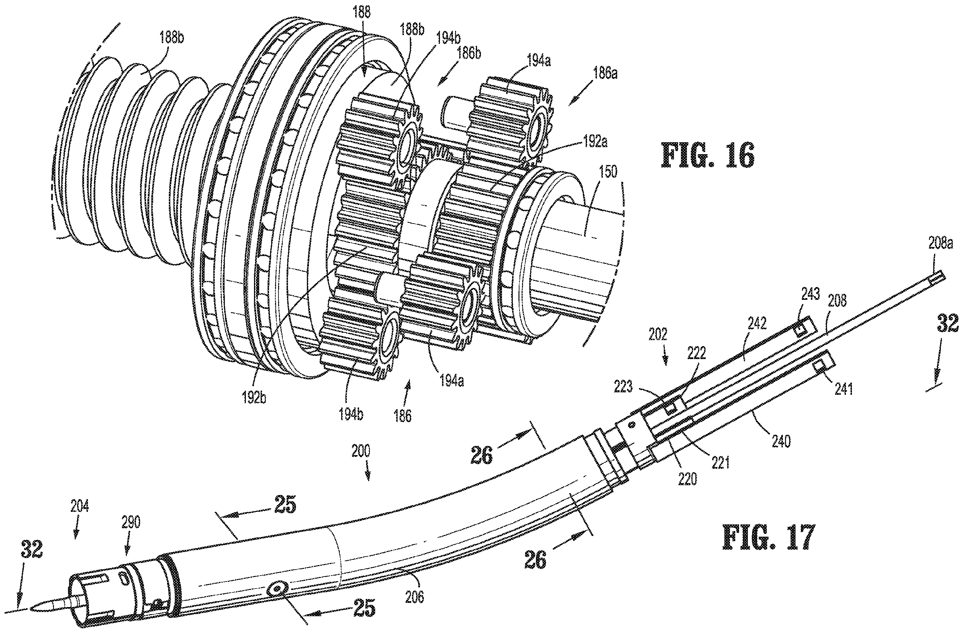

A distal end of third proximal drive shaft 120 of drive coupling assembly 110 includes a geared portion 146a that engages a geared portion 146b formed on a proximal end of drive member 140 of drive transfer assembly 130. The size of geared portion 146a on third proximal drive shaft 120 and geared portion 146b on drive member 140 are the same size to provide a 1:1 gear ratio between third proximal drive shaft 120 and drive member 140. In this manner, third proximal drive shaft 120 and drive member 140 rotate at the same speed. However, it is envisioned that either or both of geared portions 146a, 146b may be of different sizes to alter the gear ratio between third proximal drive shaft 120 and drive member 140. A distal end of drive member 140 defines a socket 145 that receives a proximal end 108a of shaft 108. Alternatively, socket 145 may be configured to operably engage a proximal end 208a of a drive shaft (FIG. 17) of an extension assembly 200 (FIG. 17).

Drive transfer assembly 130 also includes a drive connector 148 (FIG. 11) operably connecting first rotatable distal drive shaft 136 to first pusher assembly 160 and a tubular connector 150 operably connecting second rotatable distal drive shaft 138 to second pusher assembly 180. In particular, a distal end of first rotatable distal drive shaft 136 includes a geared portion 152a that engages a geared portion 152b of drive connector 148. A distal end of second rotatable distal drive shaft 138 includes a geared portion 154a that engages a drive gear 154b secured to a distal end of tubular connector 150.

As shown in FIG. 10, geared portion 152a of first rotatable distal drive shaft 136 is smaller than geared portion 152b of drive connector 148 to provide a gear ratio of greater than 1:1 between first rotatable distal drive shaft 136 and drive connector 148. In this manner, drive connector 148 rotates at a slower speed than first rotatable distal drive shaft 136. Similarly, geared portion 154a of second rotatable distal drive shaft 138 is smaller than drive gear 154b on tubular connector 150 to provide a gear ratio of greater than 1:1 between second rotatable distal drive shaft 138 and drive connector 148. In this manner, tubular connector 150 rotates at a slower speed than second rotatable distal drive shaft 138. However, it is envisioned that each of paired geared portion 152a and geared portion 152b, and geared portion 154a and drive gear 154b may be the same size to provide a gear ratio of 1:1 between respective first rotatable distal drive shaft 136 and drive connector 148 and between second rotatable distal drive shaft 138 and tubular connector 150.

With particular reference to FIGS. 9-13, first pusher assembly 160 includes proximal and distal housing sections 162, 164 (FIG. 11), a planetary gear assembly 166 operably mounted within proximal housing section 162, a screw member 168 (FIG. 11) operably connected to planetary gear assembly 166 and rotatably supported within distal housing section 164, and a pusher member 170 (FIG. 11) operably connected to screw member 168 and slidably disposed within distal housing section 164. Planetary gear assembly 166 includes first and second planetary gear systems 166a, 166b (FIG. 10). First planetary gear system 166a includes a central drive gear 172a mounted on a distal end of drive connector 148 of drive transfer assembly 130 and a plurality of planetary gears 174a rotatably mounted to a rotatable support ring 176.

Each planetary gear 174a of first planetary gear system 166a engages central drive gear 172a and a toothed inner surface 165 of proximal housing section 162. As central drive gear 172a rotates in a first direction, e.g., clockwise, each planetary gear 174a rotates in a second direction, e.g., counter-clockwise. As each planetary gear 174a rotates in the second direction, engagement of planetary gears 174a with toothed inner surface 165 of distal housing section 162 causes rotatable support ring 176 to rotate in the first direction. Conversely, rotation of central drive gear 172a in the second direction causes rotation of each planetary gear 174a in the first direction thereby causing rotation of rotatable support ring 176 in the second direction. The configuration of first planetary gear system 166a provides a reduction in the gear ratio. In this manner, the speed of rotation of rotatable support ring 174 is less than the speed of rotation of central drive gear 170a.

Second planetary gear system 166b includes a central drive gear 172b securely affixed to rotatable support ring 176 and a plurality of planetary gears 174b rotatably mounted to a proximal end surface 168a of screw member 168. Each planetary gear 174b of second planetary gear system 166b engages central drive gear 172b and toothed inner surface 165 of proximal housing section 162. As rotatable support ring 176 of first planetary gear system 166a rotates in the first direction thereby causing central drive gear 172b to also rotate in the first direction, each planetary gear 174b rotates in the second direction. As each planetary gear 174b rotates in the second direction, engagement of planetary gears 174b with toothed inner surface 165 of proximal housing section 162 causes screw member 168 to rotate in the first direction. Conversely, rotation of central drive gear 172b in the second direction causes rotation of each planetary gear 174b in the first direction, thereby causing screw member 168 to rotate in the second direction. The configuration of second planetary gear system 166b provides a reduction in the gear ratio. In this manner, the speed of rotation of screw member 168 is less than the speed of rotation of central drive gear 172b. First and second planetary gear systems 166a, 166b operate in unison to provide a reduction in the gear ratio between first rotatable proximal drive shaft 116 and screw member 168. In this manner, the reduction in the speed of rotation of screw member 168 relative to drive connector 148 is a product of the reduction provided by the first and second planetary gear systems 166a, 166b.

Screw member 168 is rotatably supported within proximal housing portion 162 and includes a threaded distal end 168b that operably engages a threaded inner surface 170a of pusher member 170. As screw member 168 is rotated in the first direction, engagement of threaded distal end 168b of screw member 168 with threaded inner surface 170a of pusher member 170 (which is keyed to permit axial translation and prevent rotation thereof) causes longitudinal advancement of pusher member 170, as indicated by arrows "A" in FIG. 12. Conversely, rotation of screw member 168 in the second direction causes retraction of pusher member 170.

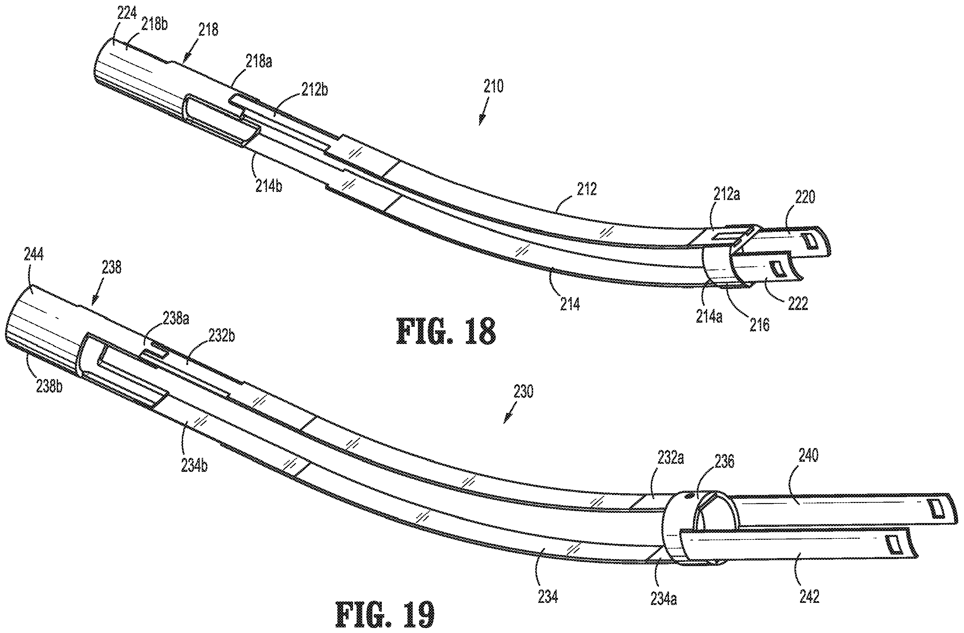

Pusher member 170 of first pusher assembly 160 of adapter assembly 100 includes a pair of tabs 178 formed on a distal end thereof for engaging connector extensions 240, 242 (FIG. 19) of outer flexible band assembly 230 (FIG. 19) of extension assembly 200 (FIG. 17). Although shown as tabs 178, it is envisioned that pusher member 170 may include any structure suitable for selectively engaging connector extensions 240, 242 of outer flexible band 230 of extension assembly 200.

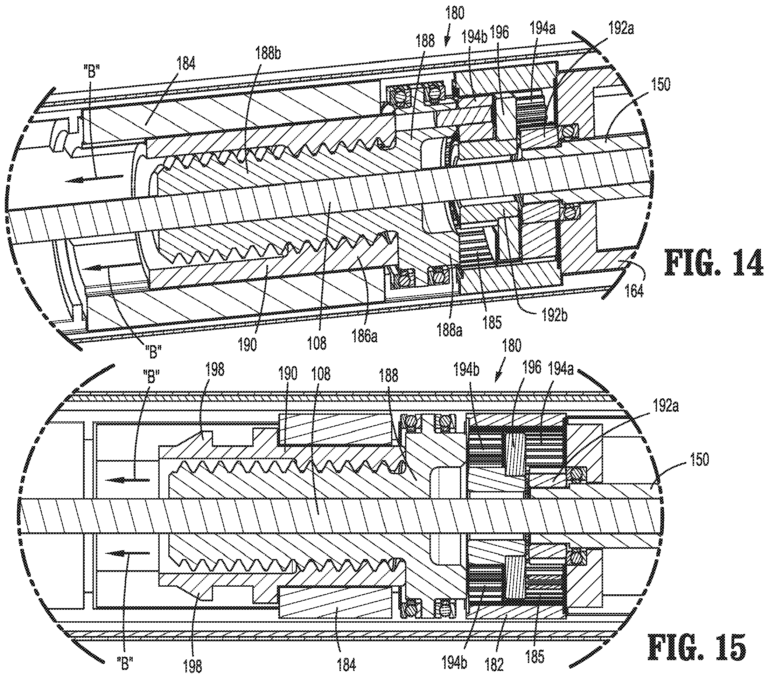

With particular reference now to FIGS. 14-16, second pusher assembly 180 is substantially similar to first pusher assembly 160, and includes proximal and distal housing sections 182, 184, a planetary gear assembly 186 operably mounted within proximal housing section 182, a screw member 188 operably connected to planetary gear assembly 186 and rotatably supported within distal housing section 184, and a pusher member 190 operably connected to screw member 188 and slidably disposed within distal housing section 184. Planetary gear assembly 186 includes first and second planetary gear systems 186a, 186b (FIG. 16). First planetary gear system 186a includes a central drive gear 192a mounted on a distal end of tubular connector 150 of drive transfer assembly 130 and a plurality of planetary gears 194a rotatably mounted to a rotatable support ring 196.

Each planetary gear 194a of first planetary gear system 186a engages central drive gear 192a and a toothed inner surface 185 of proximal housing section 182. As central drive gear 192a rotates in a first direction, e.g., clockwise, each planetary gear 194a rotates in a second direction, e.g., counter-clockwise. As each planetary gear 194a rotates in the second direction, engagement of planetary gears 194a with toothed inner surface 185 of distal housing section 182 causes rotatable support ring 196 to rotate in the first direction. Conversely, rotation of central drive gear 192a in the second direction causes rotation of each planetary gear 194a in the first direction thereby causing rotation of rotatable support ring 196 in the second direction. The configuration of first planetary gear system 186a provides a reduction in the gear ratio. In this manner, the speed of rotation of rotatable support ring 194 is less than the speed of rotation of central drive gear 190a.

Second planetary gear system 186b includes a central drive gear 192b securely affixed to rotatable support ring 196 and a plurality of planetary gears 194b rotatably mounted to a proximal end surface 188a of screw member 188. Each planetary gear 194b of second planetary gear system 186b engages central drive gear 192b and toothed inner surface 185 of proximal housing section 182. As rotatable support ring 196 of first planetary gear system 186a rotates in the first direction thereby causing central drive gear 192b to also rotate in the first direction, each planetary gear 174b rotates in the second direction. As each planetary gear 194b rotates in the second direction, engagement of planetary gears 194b with toothed inner surface 185 of proximal housing section 182 causes screw member 188 to rotate in the first direction. Conversely, rotation of central drive gear 192b in the second direction causes rotation of each planetary gear 194b in the first direction, thereby causing screw member 198 to rotate in the second direction. The configuration of second planetary gear system 186b provides a reduction in the gear ratio. In this manner, the speed of rotation of screw member 188 is less than the speed of rotation of central drive gear 182b. First and second planetary gear systems 186a, 186b operate in unison to provide a reduction in the gear ratio between second rotatable proximal drive shaft 118 and screw member 188. In this manner, the reduction in the speed of rotation of screw member 188 relative to tubular connector 150 is a product of the reduction provided by the first and second planetary gear systems 186a, 186b.

Screw member 188 is rotatably supported within proximal housing portion 182 and includes a threaded distal end 188b that operably engages a threaded inner surface 190a of pusher member 190. As screw member 188 is rotated in the first direction, engagement of threaded distal end 188b of screw member 188 with threaded inner surface 190a of pusher member 190 (which is keyed to permit axial translation and prevent rotation thereof) causes longitudinal advancement of pusher member 190. Conversely, rotation of screw member 188 in the second direction causes retraction of pusher member 190.

Pusher member 190 of second pusher assembly 180 of adapter assembly 100 includes a pair of tabs 198 formed on a distal end thereof for engaging connector extensions 220, 224 (FIG. 18) of inner flexible band assembly 220 (FIG. 18) of extension assembly 200 (FIG. 17). Although shown as tabs 198, it is envisioned that pusher member 190 may include any structure suitable for selectively engaging connector extensions 240, 242 of outer flexible band 230 of extension assembly 200.

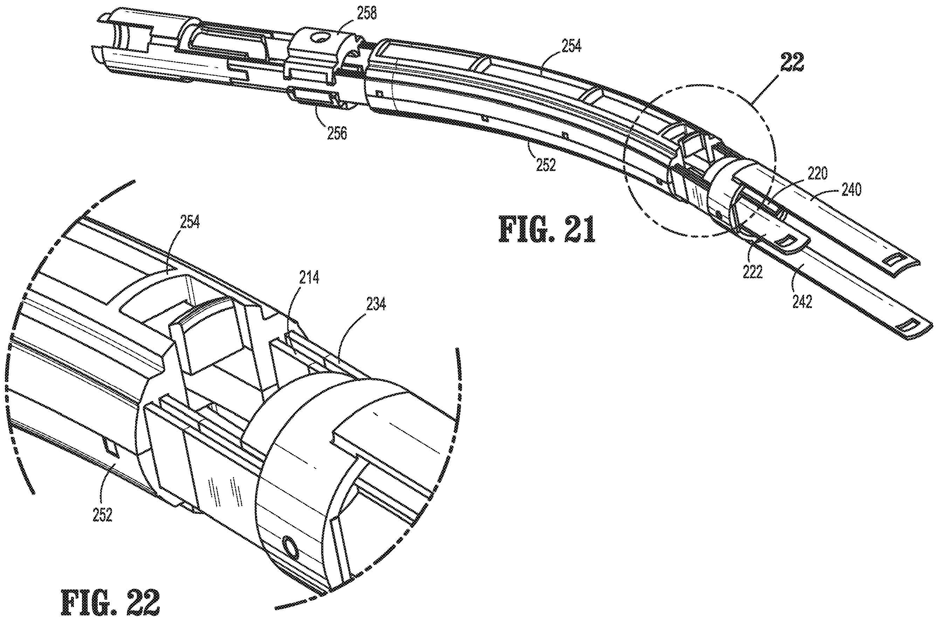

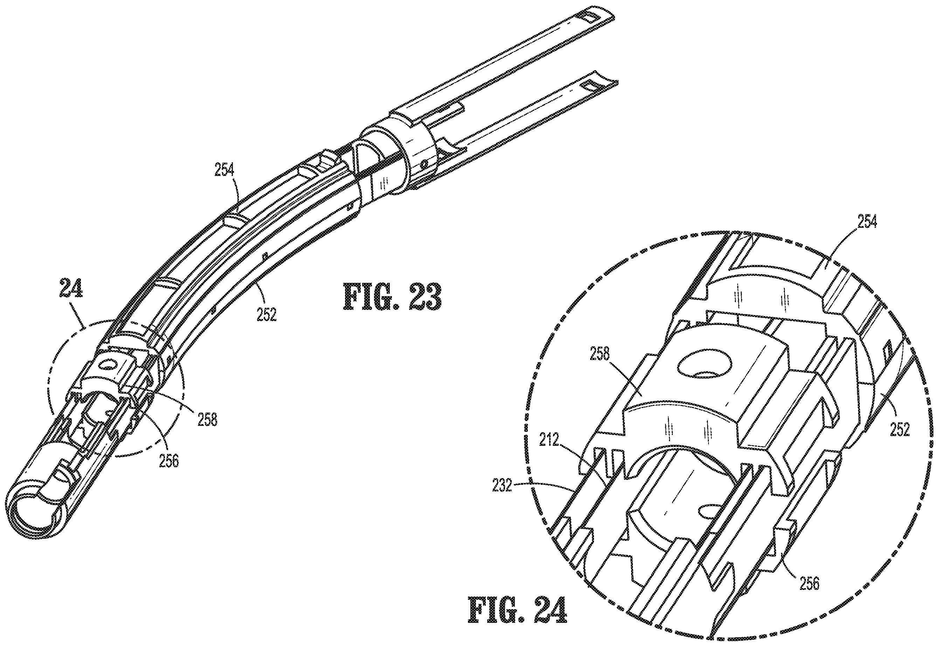

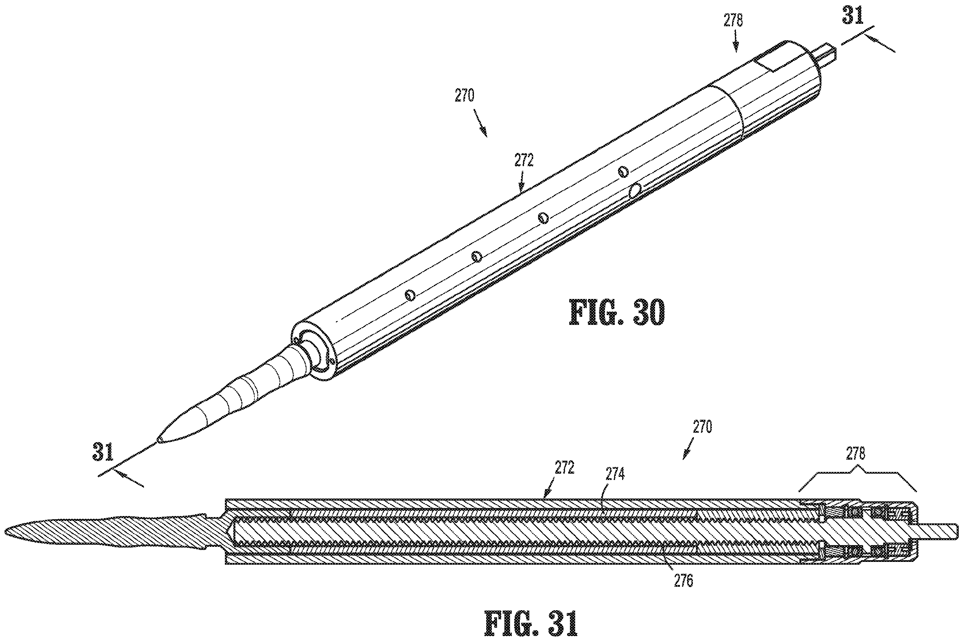

Turning now to FIGS. 17-34, extension assembly 200 for operably connecting adapter assembly 100 (FIG. 3) with a circular loading unit, e.g. loading unit 40 (FIG. 34) and an anvil assembly, e.g., anvil assembly 50 (FIG. 34) will be described. In particular, a proximal end 202 of extension assembly 200 operably connects with distal end 104 (FIG. 3) of adapter assembly 100 (FIG. 3) and a distal end 204 of extension assembly 200 operably connects with loading unit 40 and anvil assembly 50. As shown, extension assembly 200 provides a slight curvature between proximal and distal end 202, 204. In an alternative embodiment, extension assembly 200 may be straight or may include a greater curvature. In accordance with the present disclosure, extension assembly 200 may be substantially or fully rigid along its entire length.

Although extension assembly 200 will be shown and described as being used to connect loading unit 40 and anvil assembly 50 to adapter assembly 100 (FIG. 3), it is envisioned that the aspects of the present disclosure may be modified for use with various loading units, anvil assemblies, and adapter assemblies. Exemplary loading units and anvil assemblies are described in commonly owned U.S. Patent Nos. 8,590,763 and 9,579,099, and U.S. Pat. Appl. Ser. No. 14/056,301 (U.S. Patent Publication No. 2015/0108201, filed on October 17, 2013.

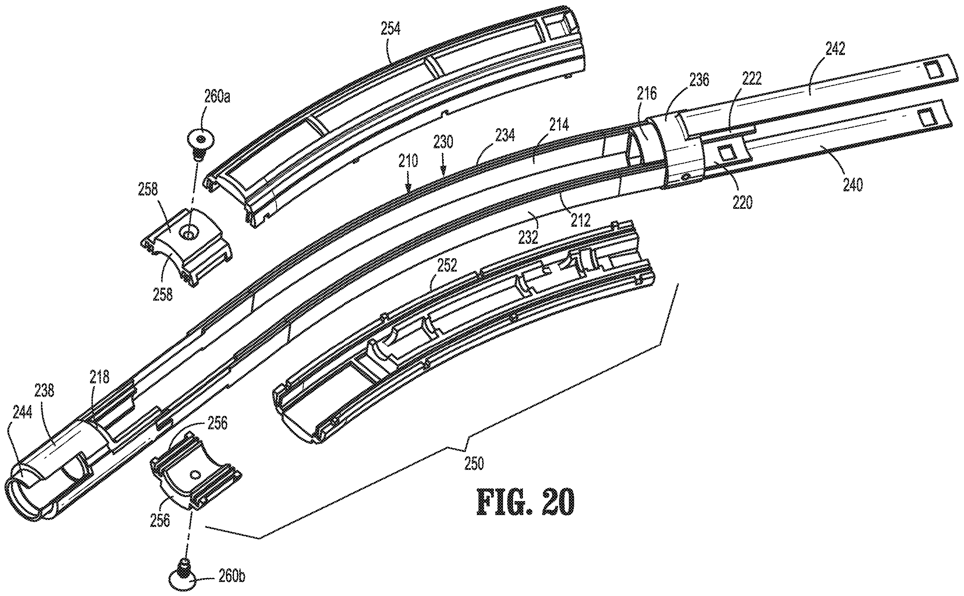

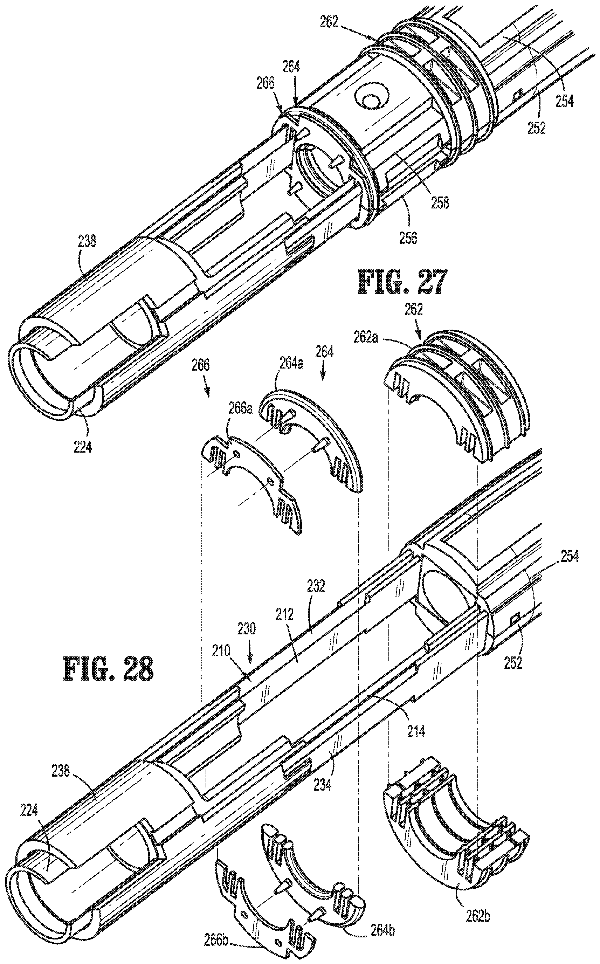

Extension assembly 200 includes an inner flexible band assembly 210 (FIG. 18), about an outer flexible band assembly 230 (FIG. 19) slidably disposed about inner flexible band assembly 210, a frame assembly 250 (FIG. 20) for supporting inner and outer flexible band assemblies 210, 230, a trocar assembly 270 (FIG. 28) operably received through inner and outer flexible band assemblies 210, 230, and a connector assembly 290 for securing loading unit 40 (FIG. 34) to extension assembly 200. An outer sleeve 206 (FIG. 17) is received about frame assembly 250 and trocar assembly 270, and inner and outer flexible band assemblies 210, 230 are slidably received through outer sleeve 206. As will be described in further detail below, extension assembly 200 may include a drive shaft 208 operably connected to trocar assembly 270 and extending through proximal end 202 of extension assembly 200.

With reference to FIG. 18, inner flexible band assembly 210 includes first and second inner flexible bands 212, 214, a support ring 216, a support base 218, and first and second connection extensions 220, 222. Proximal ends 212a, 214a of respective first and second inner flexible bands 212, 214 are laterally spaced apart and securely attached to support ring 216. Distal ends 212b, 214b of first and second inner flexible bands 212, 214 are laterally spaced apart and securely attached to a proximal end 218a of support base 218. Each of first and second inner flexible bands 212, 214 may be attached to support ring 216 and/or support base 218 in any suitable manner, including, for example, by press-fitting, welding, adhesives, and/or with mechanical fasteners. As will be described in further detail below, inner flexible band assembly 210 is configured to be slidably received about trocar assembly 270 (FIG. 28) and within outer flexible band assembly 230 (FIG. 19) and outer sleeve 206 (FIG. 17).

First and second connection extensions 220, 222 of inner flexible band assembly 210 extend proximally from support ring 216 and operably connect inner flexible band assembly 210 with pusher member 190 (FIG. 15) of second pusher assembly 180 (FIG. 15) of adapter assembly 100 (FIG. 3). In particular, each of first and second connection extensions 220, 222 define respective openings 221, 223 configured to receive tabs 198 (FIG. 15) of pusher member 190 (FIG. 15) of second pusher assembly 180. Receipt of tabs 198 of pusher member 190 within openings 221, 223 of respective first and second extensions 220, 222 secure inner flexible band assembly 210 of extension assembly 200 with second pusher assembly 180 of adapter assembly 100. First and second connection extensions 220, 222 may be integrally formed with support ring 216, or attached thereto in any suitable manner.

Support base 218 extends distally from inner flexible bands 212, 214 and is configured to selectively connect extension assembly 200 with loading unit 40 (FIG. 34). Specifically, a distal end 218a of support base 218 includes a flange 224 for operable engagement with an axially movable assembly (not shown) of loading unit 40 (FIG. 34). In one embodiment, flange 224 is configured for connection with a knife assembly (not shown) of loading unit 40 (FIG. 34).

With reference now to FIG. 19, outer flexible band assembly 230 is substantially similar to inner flexible band assembly 210 and includes first and second flexible bands 232, 234 laterally spaced and connected on proximal ends 232a, 234a to a support ring 236 and on distal ends 234b, 234b to a proximal end 238a of a support base 238. Each of first and second outer flexible bands 232, 234 may be attached to support ring 236 and support base 238 in any suitable manner, including, for example, by press-fitting, welding, adhesives, and/or with mechanical fasteners. As will be described in further detail below, outer flexible band assembly 230 is configured to receive trocar assembly 270 (FIG. 28) therethrough.

First and second connection extensions 240, 242 of outer flexible band assembly 230 extend proximally from support ring 236 and operably connect outer flexible band assembly 230 with pusher member 170 (FIG. 12) of first pusher assembly 160 (FIG. 12) of adapter assembly 100 (FIG. 1). In particular, each of first and second connection extensions 240, 242 define respective openings 241, 243 configured to receive tabs 178 (FIG. 12) of pusher member 170 of first pusher assembly 180. Receipt of tabs 178 of pusher member 170 within openings 241, 243 of respective first and second extensions 240, 242 secures outer flexible band assembly 230 of extension assembly 200 with first pusher assembly 180 of adapter assembly 100. First and second connection extensions 240, 242 may be integrally formed with support ring 236, or attached thereto in any suitable manner.

Support base 238 extends distally from outer flexible bands 232, 234 and is configured to selectively connect extension assembly 200 with loading unit 40 (FIG. 34). Specifically, a distal end 238b of support base 238 includes a flange 244 for operable engagement with an axially movable assembly (not shown) of a loading unit (not shown). In one embodiment, flange 244 is configured for connection with a staple pusher assembly (not shown) of loading unit 40 (FIG. 34).

With reference now to FIGS. 20-26, frame assembly 250 includes first and second proximal spacer members 252, 254, and first and second distal spacer members 256, 258. When secured together, first and second proximal spacer members 252, 254 define a pair of inner longitudinal slots 253a for slidably receiving first and second flexible bands 212, 214 (FIG. 18) of inner flexible band assembly 210 (FIG. 18) and a pair of outer longitudinal slots 253b for slidably receiving first and second flexible bands 232, 234 (FIG. 19) of outer flexible band assembly 230 (FIG. 19). First and second proximal spacer members 252, 254 further define a longitudinal passage 255 for receipt of trocar assembly 270.

As shown, first and second proximal spacer members 252, 254 are formed of plastic and are secured together with a snap-fit arrangement. Alternatively, first and second proximal spacer members 252, 254 may be formed of metal or other suitable material and may be secured together in any suitable manner, including by welding, adhesives, and/or using mechanical fasteners.

First and second distal spacer members 256, 258 define a pair of inner slots 257a for slidably receiving first and second flexible bands 212, 214 (FIG. 18) of inner flexible band assembly 210 (FIG. 18) and a pair of outer slots 257b for slidably receiving first and second flexible bands 232, 234 (FIG. 19) of outer flexible band assembly 230 (FIG. 19). First and second distal spacer members 256, 258 further define a longitudinal passage 259 for receipt of trocar assembly 270.

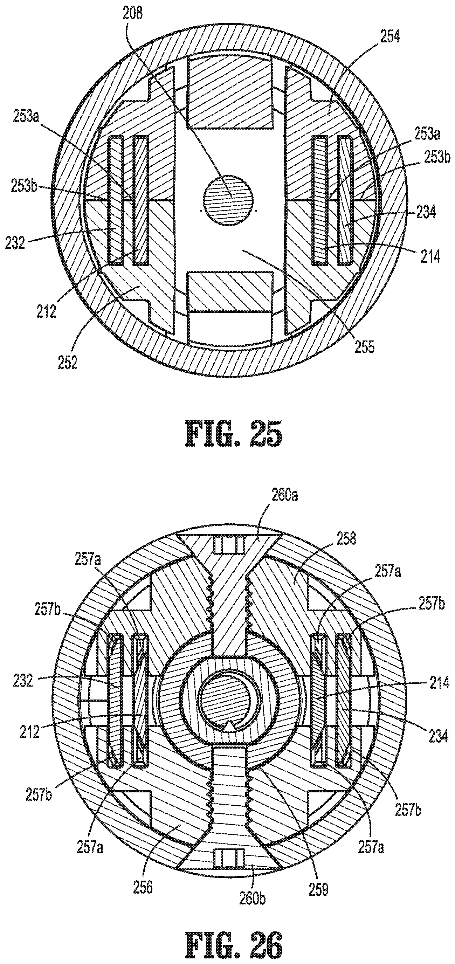

As shown, each of first and second distal spacer members 256, 258 are secured about inner and outer flexible band assemblies 210, 230 and to outer sleeve 206 (FIG. 17) by a pair of screws 260a, 260b (FIG. 26). Alternatively, first and second distal spacer members 256, 258 may be secured together in any suitable manner, including by welding, adhesives, and/or using mechanical fasteners. First and second distal spacer members 256, 258 may be formed of metal or any other suitable material.

With reference now to FIGS. 27 and 28, frame assembly 250 further includes a proximal seal member 262 and first and second distal seal members 264, 266. Each of proximal seal member 252 and first and second distal seal members 264, 266 include seals halves 262a, 262b, 264a, 264b, 266a, 266b, respectively. Proximal seal member 262 is received between first and second proximal spacer members 252, 254 and first and second distal spacer members 256, 258. First half 264a of first distal seal member 264 is secured to first half 266a of second distal seal member 266 and second half 264b of first distal seal member 264 is secured to second half of second distal seal member 266. Proximal seal member 262 and first and second distal seal members 264, 266 engage outer sleeve 206 (FIG. 17), inner and outer flexible bands 212, 214 and 232, 234 of respective inner and outer flexible band assemblies 210, 230 and trocar assembly 270 (FIG. 28) in a sealing manner. In this manner, proximal seal member 262 and first and second distal seal members 264, 266 operate to provide a fluid tight seal between distal end 204 and proximal end 202 of extension assembly 200.

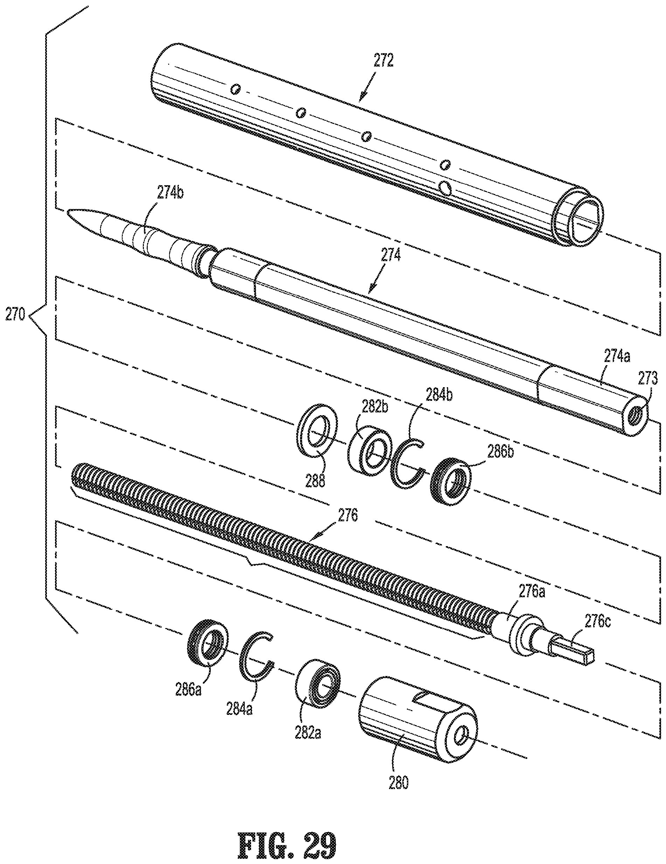

With reference to FIGS. 29-32, trocar assembly 270 of extension assembly 200 includes an outer housing 272, a trocar member 274 slidably disposed within tubular outer housing 272, and a drive screw 276 operably received within trocar member 274 for axially moving trocar member 274 relative to tubular housing 272. In particular, trocar member 274 includes a proximal end 274a having an inner threaded portion 275 which engages a threaded distal portion 276b of drive screw 276. As drive screw 276 is rotated within trocar member 274, engagement of inner threaded portion 275 of trocar member 274 with threaded distal portion 276b of drive screw 276 causes longitudinal movement of trocar member 274 within outer housing 272 of trocar assembly 270. Rotation of drive screw 276 in a first direction causes longitudinal advancement of trocar member 274 and rotation of drive screw 276 in a second direction causes longitudinal retraction of trocar member 274. A distal end 274b of trocar member 274 is configured to selectively engage anvil assembly 50 (FIG. 34).

A bearing assembly 278 is mounted to a proximal end 272a of outer housing 272 of trocar assembly 270 for rotatably supporting a proximal end 276a of drive screw 276 relative to outer housing 272 and trocar member 274. Bearing assembly 278 includes a housing 280, proximal and distal spacers 282a, 282b, proximal and distal retention clips 284a, 284b, proximal and distal bearings 286a, 286b, and a washer 288. As shown, proximal end 276a of drive screw 276 includes a flange 276c for connection with a link assembly 277. A distal portion 277b of link assembly 277 is pivotally received between first and second proximal spacer members 252, 254 and operably engages flange 276c on drive screw 276. A proximal end 277a of link assembly 277 is configured for operable engagement with a distal end 208b of drive shaft 208.

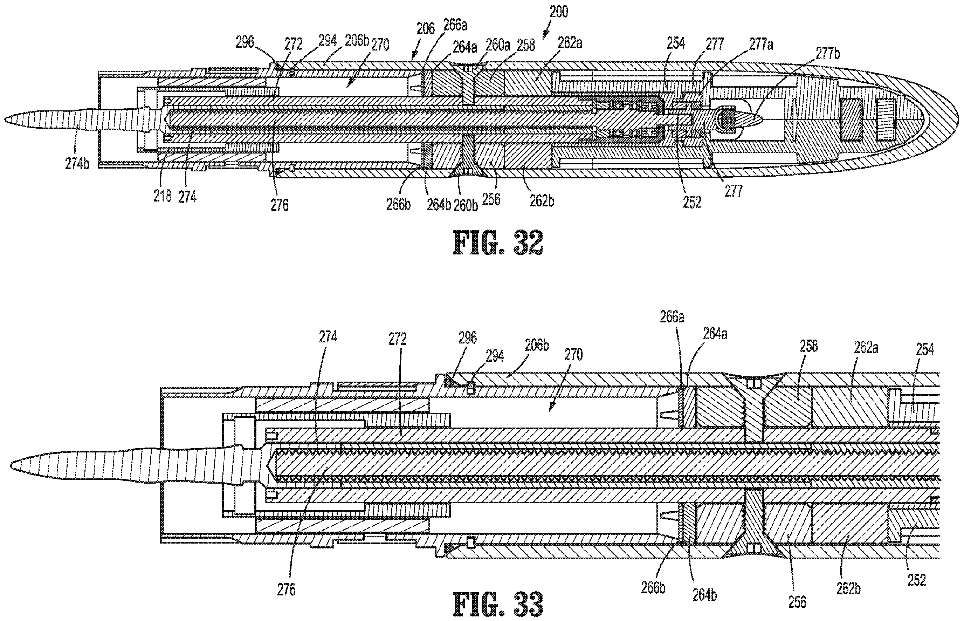

With reference now to FIGS. 32 and 33, connector assembly 290 of extension assembly 200 includes a tubular connector 292 attached to a distal end 206a of outer sleeve 206 and about distal ends of inner and outer flexible assemblies 210, 230 (FIG. 26) and trocar assembly 270. In particular, a proximal end 292a of tubular connector 292 is received within and securely attached to distal end 206b of outer sleeve 206 by a retaining clip 294. An O-ring 296 forms a fluid tight seal between tubular connector 292 of connector assembly 290 and outer sleeve 206. A distal end 292b of tubular connector 292 is configured to selectively engage a proximal end of loading unit 40 (FIG. 34). Distal end 292b of tubular connector 292 engages the circular loading unit with a snap-fit arrangement, bayonet coupling, or in another suitable manner.

With reference now to FIGS. 34 and 35, extension assembly 200 is connected to adapter assembly 100 by receiving proximal end 202 (FIG. 17) of extension assembly 200 within distal end 104 of adapter assembly 100. In particular, first and second connection extensions 220, 240, 222, 242 of respective inner and outer flexible band assemblies 210, 230 are received within sleeve 106 of adapter assembly 100 such that tabs 178 of pusher member 170 of first pusher assembly 160 of adapter assembly 100 are received within openings 241, 243 of respective first and second connection extensions 240, 242 of outer flexible band assembly 230 to secure outer flexible band assembly 230 with first pusher assembly 160 and tabs 198 of pusher member 190 of second pusher assembly 180 of adapter assembly 100 are received within openings 221, 223 of first and second connection extensions 221, 223 of inner flexible band assembly 210 to secure inner flexible band assembly 210 with second pusher assembly 180.

As noted above, adapter assembly 100 may include a drive shaft 108 (FIG. 3) that extends from distal end 104 of adapter assembly 100. Alternatively, extension assembly 200 may include a drive shaft 208 extending from proximal portion 202 of extension assembly 200. In the event both adapter assembly 100 includes drive shaft 108 and extension assembly 200 includes drive shaft 208, prior to receipt of proximal portion 202 of extension assembly 200 within distal end 104 of extension assembly 100, one of drive shaft 108, 208 must be removed from respective adapter assembly 100 and extension assembly 200. During receipt of proximal portion 202 of extension assembly 200 within distal end 102 of adapter assembly 100, either distal end 108b (FIG. 35) of drive shaft 108b (FIG. 35) engages proximal portion 277b (FIG. 35) of link assembly 277, or proximal end 208a (FIG. 17) of drive shaft 208 (FIG. 17) is received within socket 145 of drive member 140 of drive transfer assembly 130 of extension assembly 100 (FIG. 12).

After extension assembly 200 is operably engaged with adapter assembly 100, and adapter assembly 100 is operably engaged with surgical device 10 (FIG. 1), loading unit 40 (FIG. 34) of end effector 30 (FIG. 34) may be attached to connector assembly 290 of extension assembly 200 and an anvil assembly 50 (FIG. 34) may be attached to distal end 274b of trocar 274 of extension assembly 200 in a conventional manner. During actuation of loading unit 40 and anvil assembly 50, longitudinal advancement of pusher member 190 of second pusher assembly 180 of adapter assembly 100, as described above, and as indicated by arrows "C" in FIG. 35, causes longitudinal advancement of outer flexible band assembly 230 of extension assembly 200 and longitudinal advancement of pusher member 170 of first pusher assembly 160, as described above, and as indicated by arrows "D" in FIG. 35, causes longitudinal advancement of inner flexible band assembly 210. Rotation of drive shaft 108 in a first direction, as described above, and as indicated by arrow "E", causes advancement of trocar 274 of extension assembly 200. Conversely, longitudinal retraction of pusher member 190 causes longitudinal retraction of outer flexible band assembly 230, longitudinal retraction of pusher member 170 causes longitudinal retraction of inner flexible band assembly 210, and rotation of drive shaft 108 in a second direction causes retraction of trocar 274 of extension assembly 200.

Inner flexible band assembly 210 is operably connected to a knife assembly (not show) of loading unit 40 (FIG. 34) of end effector 30 (FIG. 34) attached to connection assembly 290 of extension assembly 200, outer flexible band assembly 230 is operably connected to a staple driver assembly (not shown) of loading unit 40, and trocar 274 is operably connected to anvil assembly 50 (FIG. 34) of end effector 30 (FIG. 34). In this manner, longitudinal movement of inner flexible band assembly 210 causes longitudinal movement of the knife assembly, longitudinal movement of outer flexible band assembly 230 causes longitudinal movement of the staple driver assembly, and longitudinal movement of trocar 274 causes longitudinal movement of anvil assembly 50 relative to loading unit 40.

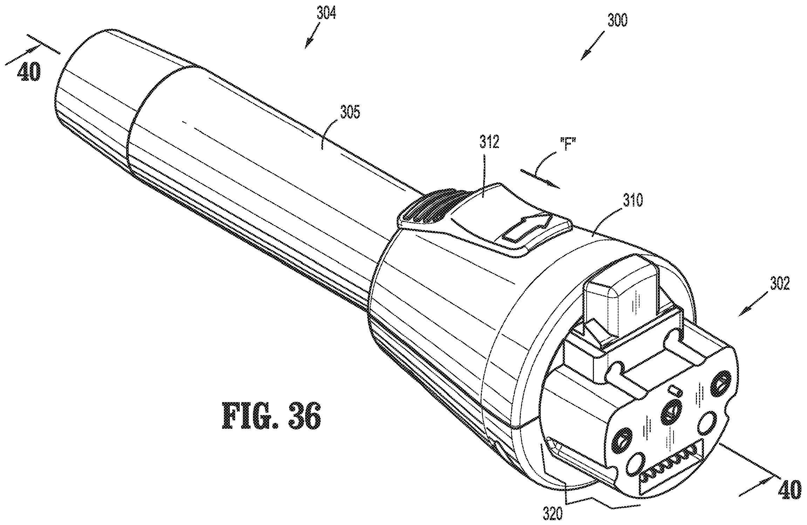

With reference to FIGS. 36-41, an adapter assembly is shown as adapter assembly 300. Adapter assembly 300 is substantially similar to adapter assembly 100 described hereinabove and will only be described as it relates to the differences therebetween.

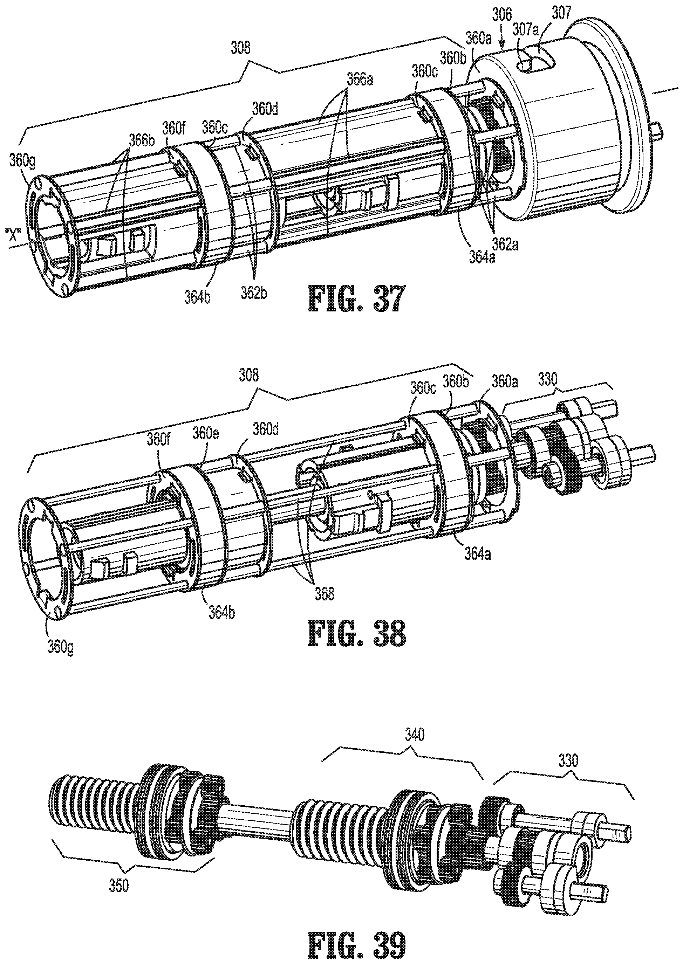

As will become apparent from the following description, the configuration of adapter assembly 300 permits rotation of a distal portion 304 of adapter assembly 300 about a longitudinal axis "X" (FIG. 37), relative to a proximal portion 302 of adapter assembly 300. In this manner, an end effector, e.g. end effector 30 (FIG. 34) secured to distal portion 304 of adapter assembly 300 or an end effector secured to an extension assembly, e.g., extension assembly 200 (FIG. 17) which is secured to distal portion 304 of adapter assembly 300 is rotatable about longitudinal axis "X" independent of movement of the surgical device (not shown) to which adapter assembly 300 is attached.

Adapter assembly 300 includes a base 306 and a support structure 308 rotatable relative to base 306 along longitudinal axis "X" of adapter assembly 300. A rotation handle 310 is rotatably secured to base 306 and fixedly secured to a proximal end of support structure 308. Rotation handle 310 permits longitudinal rotation of distal portion 304 of adapter assembly 300 relative to proximal end 302 of adapter assembly 300. As will be described in further detail below, a latch 312 is mounted to rotation handle 310 and selectively secures rotation handle 310 in a fixed longitudinal position.

Proximal portion 302 of adapter assembly 300 includes a drive coupling assembly 320 and a drive transfer assembly 330 operably connected to drive coupling assembly 320. Distal portion 304 of adapter assembly 300 includes a first pusher assembly 340 operably connected to drive transfer assembly 330, and a second pusher assembly 350 operably connected to drive transfer assembly 330. Drive coupling assembly 320 and drive transfer assembly 330 are mounted within base 306, and thus, remain rotationally fixed relative to the surgical device (not shown) to which adapter assembly 300 is attached. First pusher assembly 340 and second pusher assembly 350 are mounted within support structure 308, and thus, are rotatable relative to the surgical device (not shown) to which adapter assembly 300 is attached.

Drive coupling assembly 320 is configured to selectively secure adapter assembly 300 to a surgical device (not shown). For a detailed description of an exemplary surgical device and drive coupling assembly, please refer to commonly owned U.S. Patent Application Serial No. 14/550,183, filed November 21, 2014 (now U.S. Patent Publication No. 2015/0157321), and U.S. Patent Application Serial No. 14/822,970, filed August 11, 2015 (now U.S. Patent Publication No. 2015/0342603).

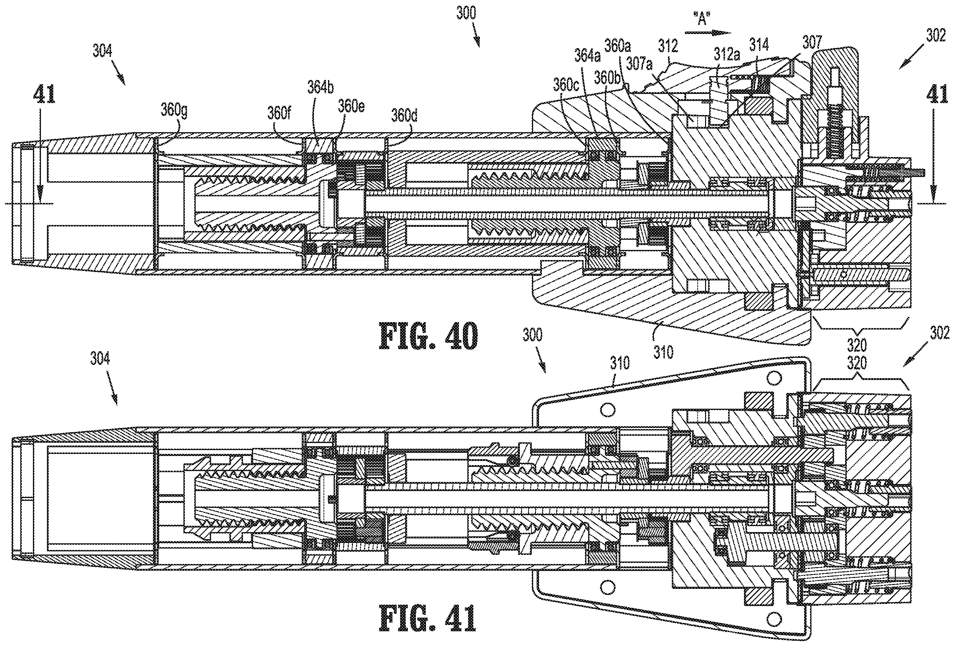

Rotation knob 310 is rotatably secured to base 306. Latch 312 includes a pin 312a (FIG. 40) configured to lock rotation knob 310 relative to base 306. In particular, pin 312a of latch 312 is received within a slot 307 formed in base 306 and is biased distally by a spring 314 into a notch 307a (FIG. 40) formed in base 306 and in communication with slot 307 to lock rotation knob 310 relative to base 306. Proximal movement of latch 312, as indicated by arrow "F" in FIG. 36, retracts pin 312a from within notch 307a to permit rotation of rotation knob 310 relative to base 306. Although not shown, it is envisioned that base 306 may define a number of notches radially spaced about base 306 and in communication with slot 307 that permit rotation knob 310 to be locked in a number of longitudinal orientations relative to base 306.

Drive transfer assembly 330, first drive pusher assembly 340, and second drive pusher assembly 350 of adapter assembly 300 are substantially identical to respective drive transfer assembly 130, first drive pusher assembly 160, and second drive pusher assembly 180 of adapter assembly 100 described hereinabove, and therefore, will only be described as relates to the differences therebetween.

Support structure 308 is fixedly received about first and second drive pusher assemblies 340, 350 and rotatably relative to base 306. As noted above, rotation knob 310 is fixedly secured to the proximal end of support structure 308 to facilitate rotation of support structure 308 relative to base 306. Support structure 308 is retained with outer sleeve 305 of adapter assembly 300 and is configured to maintain axial alignment of first and second drive pusher assemblies 340, 350. Support structure 308 may also reduce the cost of adapter assembly 300 when compared to the cost of adapter assembly 100.

Support structure 308 respectively includes first, second, third, fourth, fifth, sixth, and seventh plates 360a, 360b, 360c, 360d, 360e, 360f, 360g, a first and a second plurality of tubular supports 362a, 362b, first and second support rings 364a, 364b, a first and a second plurality of ribs 366a, 366b, and a plurality of rivets 368. From proximal to distal, first and second plates 360a, 360b are maintained in spaced apart relation to each other by the first plurality of tubular supports 362a, second and third plates 360b, 360c are maintained in spaced apart relation to each other by first support ring 364a, third and fourth plates 360c, 360d are maintained in spaced apart relation to each other by the first plurality of support ribs 366a, fourth and fifth plates 360d, 360e are maintained in spaced apart relation to each other by the second plurality of tubular supports 362b, fifth and sixth plates 360e, 360f are maintained in spaced apart relation to each other by second support ring 364b, and sixth and seventh plates 360f, 360g are maintained in spaced apart relation to each other by the second plurality of support ribs 366b. First, second, third, fourth, fifth, sixth, and seventh plates 360a-g are held together by a plurality of rivets 368 secured to first and seventh plates 360a, 360g and extending through second, third, fourth, fifth, and sixth plates 360b-360f, first and second support rings 364a, 364b, and respective first and second plurality of tubular support 362a, 362b.

Adapter assembly 300 operates in a substantially similar manner to adapter assembly 100 described hereinabove. In addition, as described in detail above, adapter assembly 300 is configured to permit rotation of an end effector, e.g., end effector 30 (FIG. 34) attached to adapter assembly 300 or attached to an extension assembly that is attached to adapter assembly 300 to be selectively rotated about longitudinal axis "X" (FIG. 37) during use.

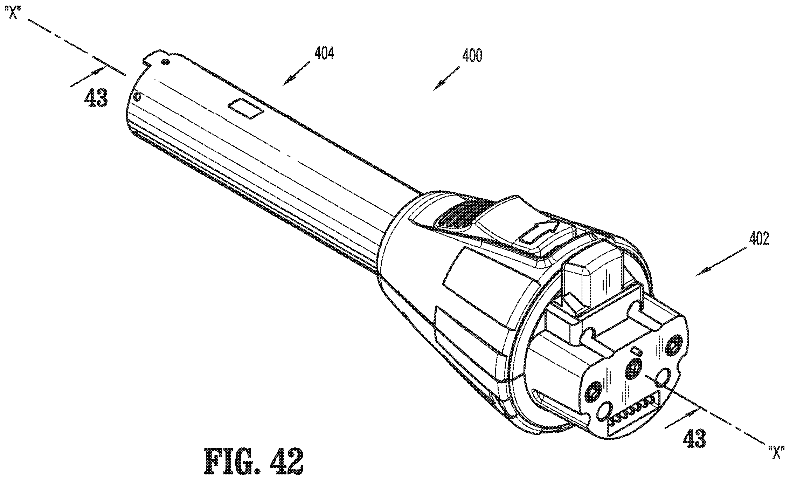

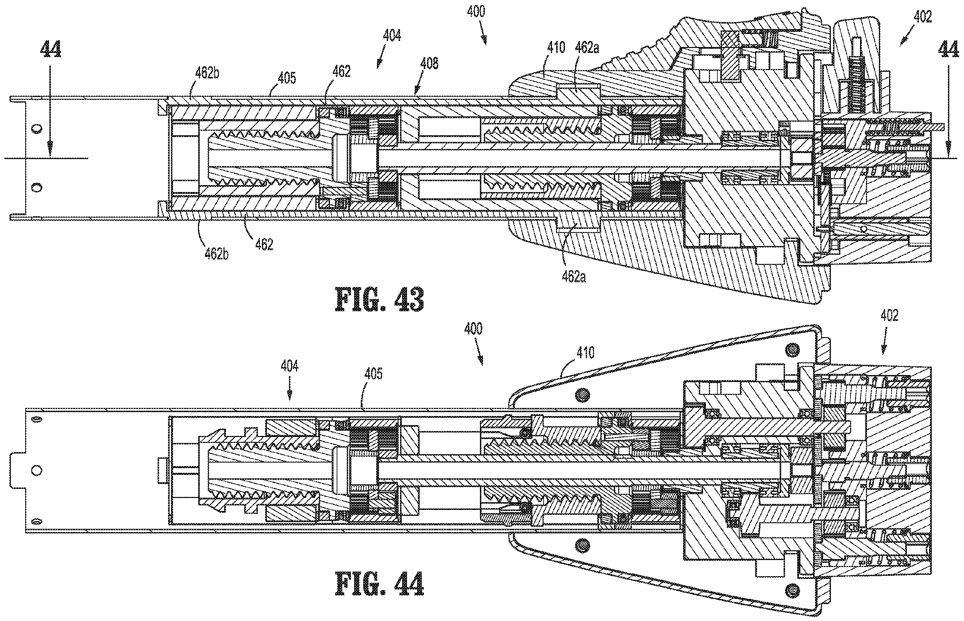

With reference now to FIGS. 42-44, an adapter assembly is shown generally as adapter assembly 400. Adapter assembly 400 is substantially similar to adapter assemblies 100 and 300 described hereinabove, and therefore will only be described as relates to the differences therebetween.

Adapter assembly 400 includes a proximal portion 402 and a distal portion 404 rotatable along a longitudinal axis "X" relative to proximal portion 402. Distal portion 404 includes a support structure 408 secured to outer sleeve 405 and formed about first and second pusher assemblies 440, 450. Support structure 408 includes a plurality of reinforcing members 462 extending substantially the length of outer sleeve 405. Reinforcing members 462 each include a proximal tab 462a and a distal tab 462b which extend through outer sleeve 405 to secure reinforcing member 462 within outer sleeve 405. Proximal tabs 462 of reinforcing members 462 are further configured to engage a rotation knob 410 of adapter assembly 400. Adapter assembly 400 may include annular plates (not shown) positioned radially inward of reinforcing members 462 that maintain proximal and distal tabs 462a, 462b of reinforcing members 462 in engagement with outer sleeve 405. The annular plates may also provide structure support to distal portion 404 of adapter assembly 400.

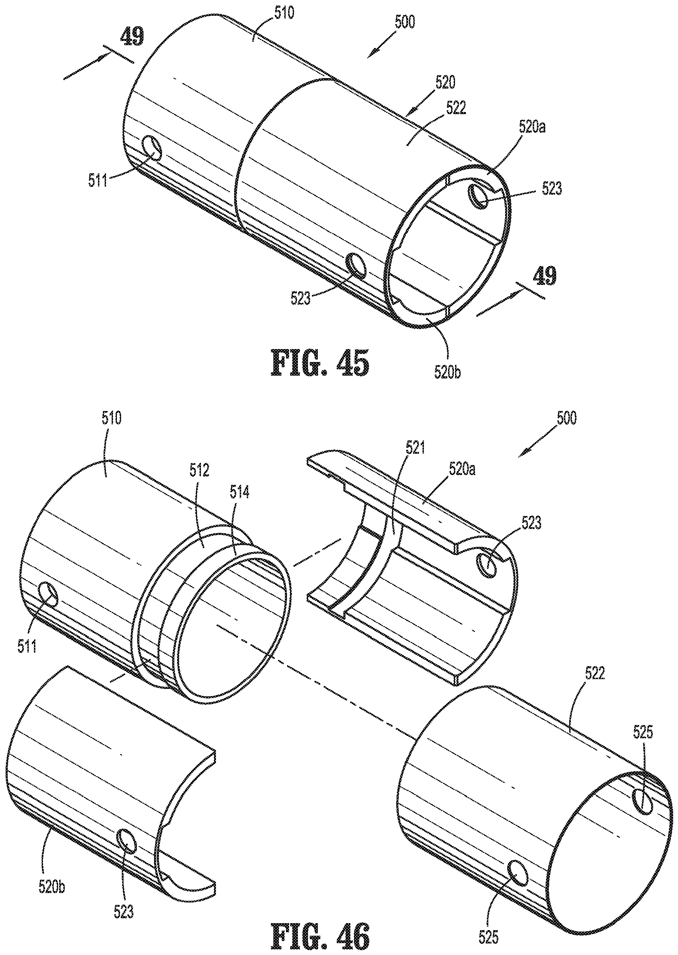

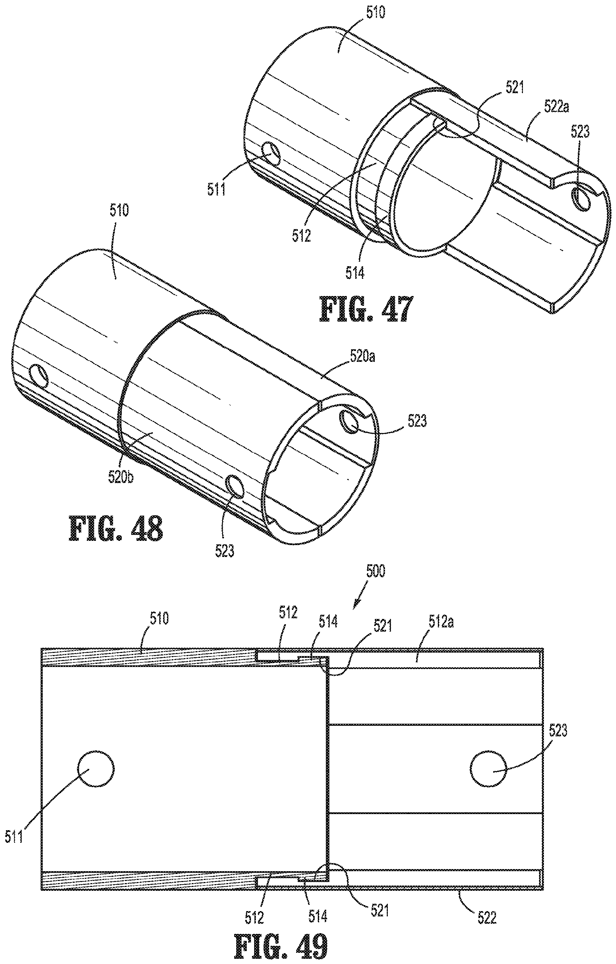

With reference to FIGS. 45-49, a connection assembly is shown generally as connection assembly 500. As shown and will be described, connection assembly 500 is configured to be attached to first and second tubular bodies (not shown) for connecting the first tubular body, e.g., adapter assembly 100 (FIG. 3), 300 (FIG. 36), 400 (FIG. 42), to the second tubular body, e.g., extension assembly 200 (FIG. 17). It is envisioned, however, that the aspects of the present disclosure may be incorporated directly into the first and second tubular bodies to permit connection of the first tubular body directly to the second tubular body.

Connection assembly 500 includes a tubular base 510 and a tubular extension 520 formed of first and second sections 520a, 520b and an outer sleeve 522. As shown, tubular base 510 defines a pair of openings 511 for securing tubular base 510 to a first tubular body (not shown). Alternatively, tubular base 510 may include only a single opening, one or more tabs (not shown), and/or one or more slots (not shown), for securing tubular base 510 to the first tubular body (not shown). A flange 512 extends from a first end of tubular base 510 and includes an annular rim 514 extending thereabout.

First and second sections 520a, 520b of tubular extension 520 are substantially similar to one another and each define an annular groove 521 formed along an inner first surface thereof. Each of first and second section 520a, 520b of tubular extension 520 is configured to be received about flange 512 of tubular base 510 such that rim 514 of tubular base 510 is received within grooves 521 of first and second sections 520a, 520b of tubular extension 520. Once first and second sections 520a, 520b of tubular extension 520 are received about flange 512 of tubular base 510, outer sleeve 522 of tubular extension 520 is received about first and second sections 520a, 520b of tubular extension 520 to secure tubular extension 520 to tubular base 510.

As shown, each of first and second sections 520a, 520b of tubular extension 520 define an opening 523 configured to be aligned with a pair of openings 525 in outer sleeve 522 to secure outer sleeve 522 to first and second sections 520a, 520b. Either or both of first and second sections 520a, 520b and outer sleeve 522 may include one or more tabs, and/or one or more slots for securing outer sleeve 522 about first and second extensions. Alternatively, outer sleeve 522 may be secured to first and second sections 520a, 520b in any suitable manner.

Outer sleeve 522 may be selectively secured about first and second extensions for selective removal of outer sleeve 522 from about first and second sections 520a, 520b to permit separation of tubular extension 520 from tubular base 510. Alternatively, outer sleeve 522 may be permanently secured about first and second sections 520a, 520b to prevent tubular extension 520 from being separated from tubular base 510. As noted above, although tubular base 510 and tubular extension 520 are shown and described as forming an independent connection assembly 500, it is envisioned that tubular base 510 may be formed on a first tubular member, e.g., adapter assembly 100 (FIG. 3) and tubular extension 520 may be formed on a second tubular member, e.g., extension assembly 200 (FIG. 17) such that the first tubular member may be directly connected to the second tubular member.

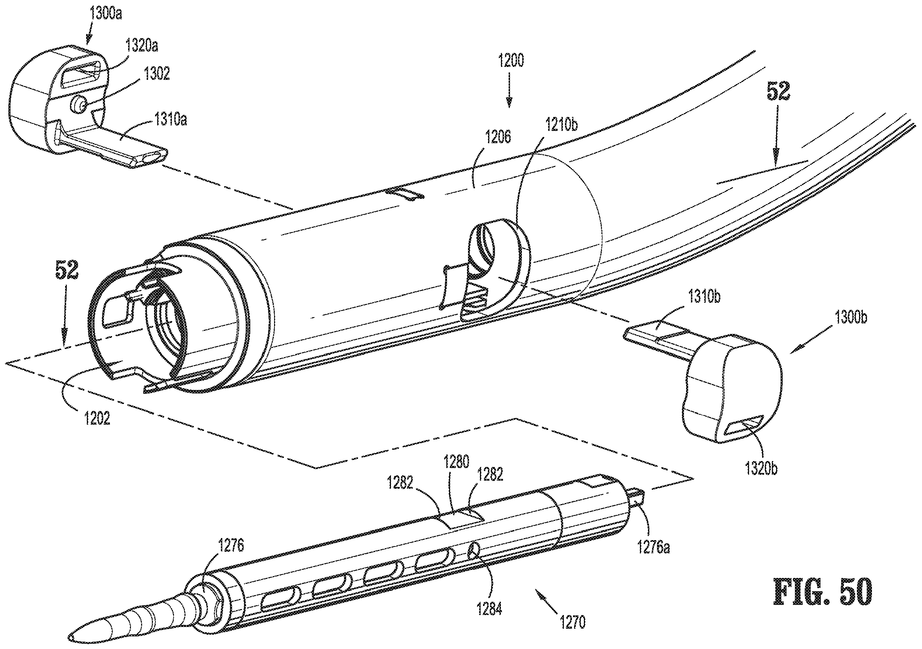

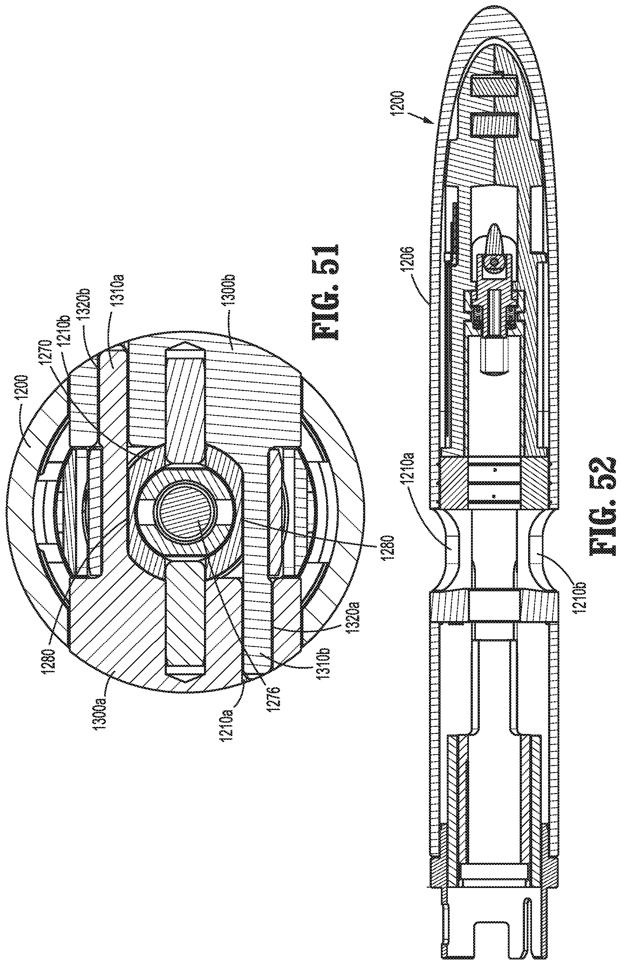

With reference to FIGS. 50-52, a trocar assembly 1270 is shown in combination with an alternate embodiment of an extension assembly 1200. Trocar assembly 1270 is similar to trocar assembly 270 described above, and not all similarities will be discussed herein. However, while trocar assembly 270 is configured for secure engagement to link assembly 277 of extension assembly 200, trocar assembly 1270 is configured for releasable engagement with extension assembly 1200.

With particular reference to FIG. 50, trocar assembly 1270 includes a pair of flattened portions 1280 about its perimeter, and extension assembly 1200 includes a pair of openings 1210a, 1210b through its outer wall or sleeve 1206 (opening 1210a is not visible in FIG. 50). When trocar assembly 1270 is engaged with extension assembly 1200, flattened portions 1280 of trocar assembly 1270 are axially aligned with openings 1210a, 1210b of extension assembly 1200. In this position, a pair of retention members 1300a, 1300b is insertable through respective openings 1210a, 1210b and adjacent (e.g., in contact with) flattened portions 1280.

More particularly, each retention member 1300a, 1300b includes an extension portion 1310a, 1310b and a receptacle 1320a, 1320b, respectively. Each extension portion 1310a, 1310b is configured to releasably engage receptacle 1320a, 1320b of the opposite retention member 1300a, 1300b. That is, extension portion 1310a of retention member 1300a is configured to releasably engage receptacle 1320b of retention member 1300b; extension portion 1310b of retention member 1300b is configured to releasably engage receptacle 1320a of retention member 1300a. It is envisioned that extension portions 1310a, 1310b respectively engage receptacles 1320b, 1320a via a snap-fit connection. It is further envisioned that retention member 1300a is identical to retention member 1300b, which may be helpful to minimize manufacturing costs and to facilitate assembly.

In use, to engage trocar assembly 1270 with extension assembly 1200, trocar assembly 1270 is inserted through a distal opening 1202 of extension assembly 1200 until a proximal end 1276a of a drive screw 1276 of trocar assembly 1200 engages a link assembly of trocar assembly 1200 (see link assembly 277 of trocar assembly 270 in FIG. 32, for example). Next, extension portion 1310a, 1310b of each retention member 1300a, 1300b, respectively, is inserted through respective opening 1210a, 1210b of outer sleeve 1206, across flattened portion 1280 of trocar assembly 1270 and into receptacle 1320b, 1320a of the other retention member 1300b, 1300a, respectively. That is, extension portion 1310a of retention member 1300a is inserted through opening 1210a (or 1210b) of outer sleeve 1206, across flattened portion 1280 and into receptacle 1320b of retention member 1300b, and extension portion 1310b of retention member 1300b is inserted through opening 1210b (or 1210a) of outer sleeve 1206, across flattened portion 1280 and into receptacle 1320a of retention member 1300a. The engagement between extension portion 1310a, flattened portion 1280 and receptacle 1320b, and the engagement between extension portion 1310b, flattened portion 1280 and receptacle 1320a is configured to prevent longitudinal translation of a trocar member 1274 of trocar assembly 1270 with respect to outer sleeve 1206 of trocar assembly 1200 (e.g., due to the engagement between extension portions 1310a, 1310b and walls 1282 of flattened portion 1280). Additionally, the engagement between extension portion 1310a, flattened portion 1280 and receptacle 1320b, and the engagement between extension portion 1310b, flattened portion 1280 and receptacle 1320a is configured to prevent relative rotation between trocar member 1274 of trocar assembly 1270 and outer sleeve 1206 of trocar assembly 1200.

Additionally, and with particular reference to FIG. 50, each retention member 1300a, 1300b includes a nub 1302 (only nub 1302 associated with retention member 1300a is shown), which is configured to mechanically engage a detent 1284 of trocar assembly 1270. It is envisioned that the engagement between nubs 1302 and detents 1284 helps maintain the proper alignment and/or orientation between retention members 1300a, 1300b and trocar assembly 1270.

To disengage retention members 1300a, 1300b from each other, it is envisioned that a user can use a tool (e.g., a screwdriver-type tool) to push extension portions 1310a, 1310b out of receptacles 1320b, 1320a, respectively. It is also envisioned that retention members 1300a, 1300b are configured to be tool-lessly (e.g., without a tool) disengaged from each other and from trocar assembly 1270. Disengagement of retention members 1300a, 1300b allows trocar assembly 1270 to be removed from outer sleeve 1206 of trocar assembly 1200 (e.g., for replacement or cleaning). It is envisioned that cleaning can occur by inserting a cleaning device at least partially within at least one opening 1210a, 1210b of outer sleeve 1206 of extension assembly 1200, and directing a cleaning fluid (e.g., saline) proximally and/or distally to help flush out any contaminants that may be present within outer sleeve 1206, for example.

Additionally, while extension assembly 1200 and trocar assembly 1270 are shown used in connection with adapter assembly 100, the present disclosure also envisions the use of extension assembly 1200 and/or trocar assembly 1270 with a surgical instrument (e.g., a circular stapling instrument) without the use of an adapter assembly.

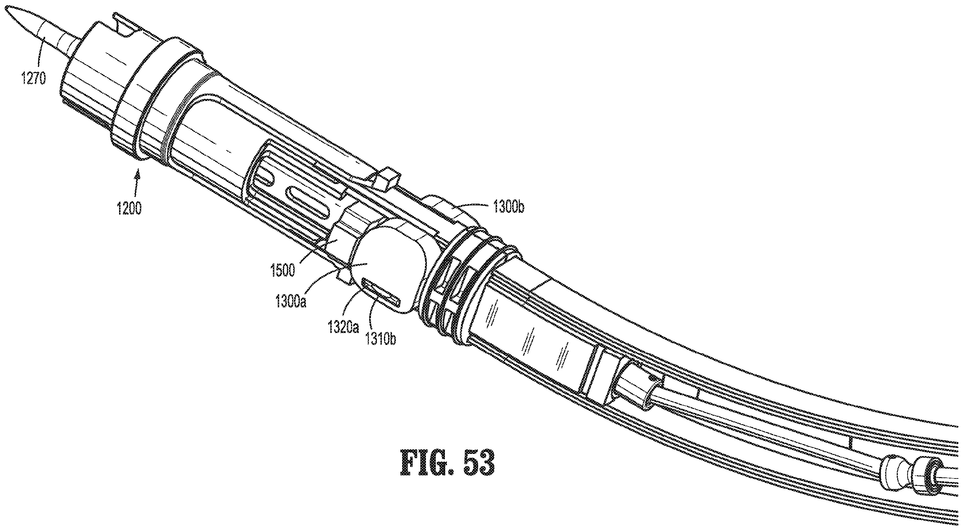

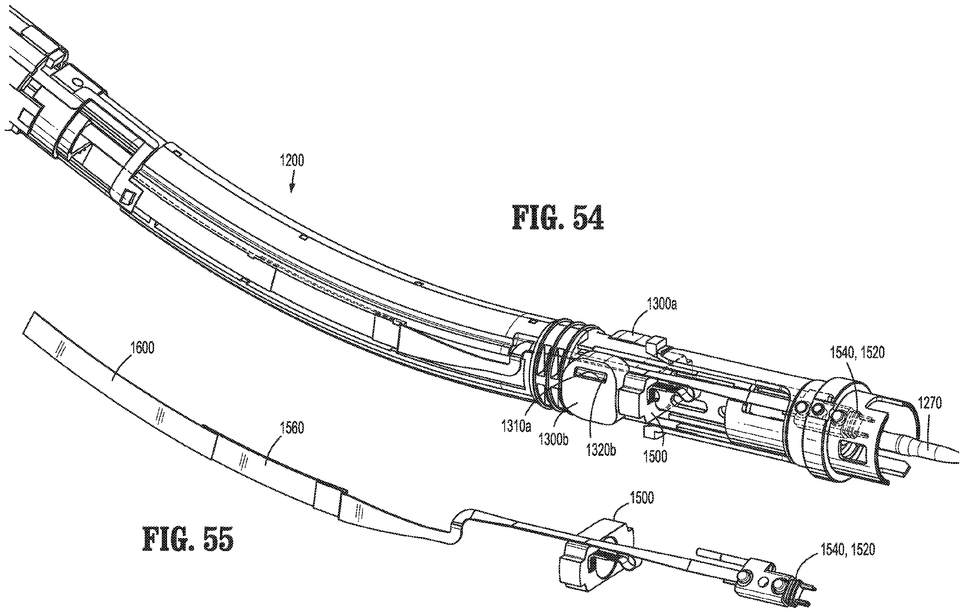

With reference to FIGS. 53-55, the present disclosure also includes a strain gauge 1500, a position sensor 1520, and a memory sensor 1540 (e.g., an E-PROM (erasable programmable read-only memory) sensor). With particular reference to FIG. 55, it is envisioned that a flexible cable 1600 extends between strain gauge 1500, position sensor 1520, memory sensor 1540 and a printed circuit board (not shown), and from the printed circuit board to an electrical connector disposed at proximal portion 302 of adapter assembly 300, for example.

It is envisioned that strain gauge 1500 is used to detect an axial load exerted on the tissue during clamping of tissue. Here, it is envisioned that if this load is too great, or exceeds a predetermined value, the user (or stapling device 10 itself) may abort the stapling operation or may choose to use a different stapling device 10 or adapter assembly 100, for example.

It is envisioned that position sensor 1520 is used to detect the axial position of the fasteners during the stapling process (e.g., when the fasteners are being ejected from adapter assembly 100). It is further envisioned that memory sensor 1540 is configured to recognize the size and/or type of staple cartridge that is engaged with adapter assembly 100 that is engaged with stapling device 10 and to relay this information to handle housing 12 of stapling device 10.

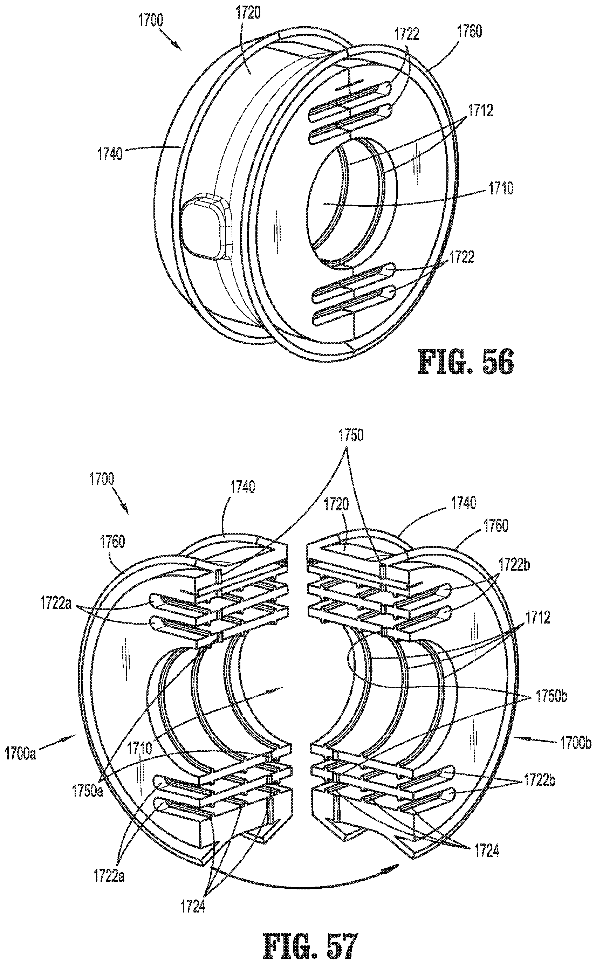

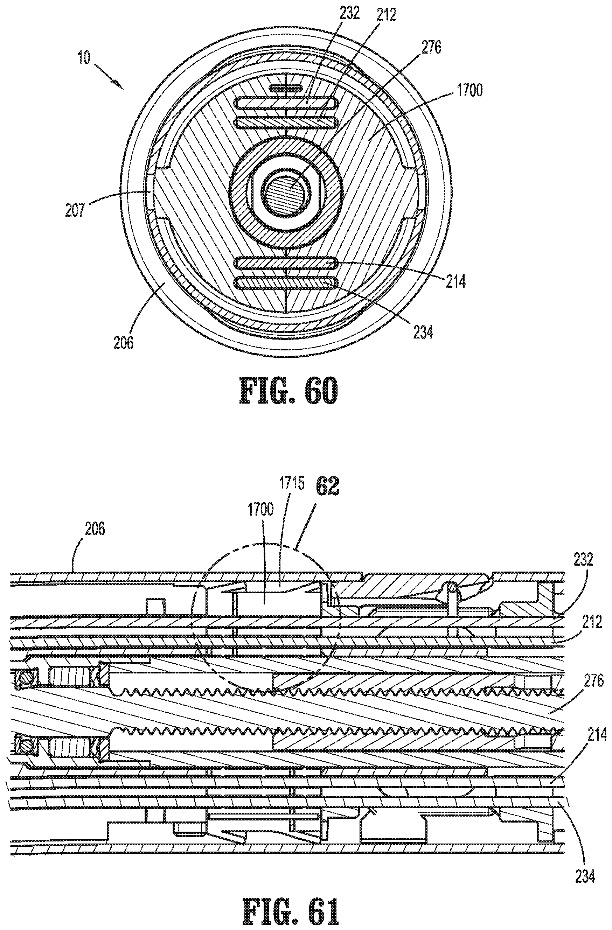

Referring now to FIGS. 56-62, a seal assembly 1700 for use with surgical device 10, adapter assembly 100, and/or extension assembly 200 is shown. Seal assembly 1700 is configured to facilitate thoroughly cleaning debris (e.g., surgical debris) from surgical device 10 following use, prior to use, and/or prior to reuse, for instance. More specifically, seal assembly 1700 is particularly useful when internal portions of surgical device 10 are flushed with a fluid to help remove debris from within surgical device 10. Further, seal assembly 1700 is configured to minimize flow traps which may occur when a flushing introduction point is located distally of a seal or seal assembly, for instance. Additionally, while seal assembly 1700 is shown and described for use a particular type of surgical device 10, seal assembly 1700 is usable with various types of surgical instruments (e.g., reusable) where cleaning and/or sterilization may be desired. Further, when used with surgical device 10 of the present disclosure, seal assembly 1700 replaces proximal seal member 262, and first and second distal seal members 264, 266 (FIGS. 27 and 28).

Seal assembly 1700 is positioned within outer sleeve 206 and defines an aperture 1710 through which an actuation member, e.g., drive screw 276, is positioned. With particular reference to FIGS. 56 and 57, seal assembly 1700 is formed of a first portion 1700a and a second portion 1700b, which are configured to engage each other (e.g., frictionally held together by an inner wall 206c of outer sleeve 206). It is envisioned that first portion 1700a is a mirror image or a substantial mirror image of second portion 1700b.

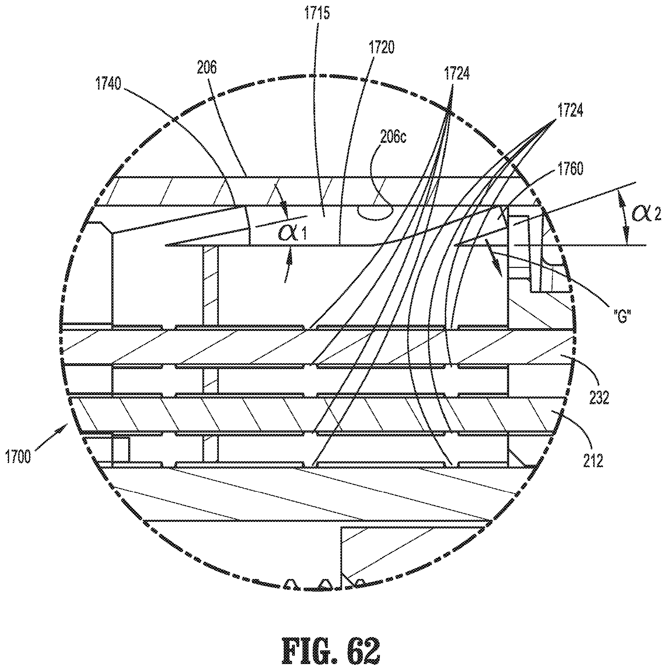

With continued reference to FIGS. 56, 57 and 62, seal assembly 1700 includes an annular body portion 1720, an annular proximal seal 1740 (e.g., a wiper seal), and an annular distal seal (e.g., a wiper seal) 1760. As shown, proximal seal 1740 and distal seal 1760 extend radially outward from body portion 1720, and define acute angles α1 and α2 (see FIG. 62), respectively, with respect to body portion 1720. Angles α1 and α2 may be the same or different from each other, and may be from about 15° to about 45°, for example. Additionally, as shown in FIG. 62, proximal seal 1740 and distal seal 1760 are configured to contact inner wall 206c of outer sleeve 206 of surgical device 10.

Body portion 1720 of seal assembly 1700 includes a plurality of channels 1722 formed therein. Channels 1722 are configured to allow inner flexible band assembly 210 (including first and second inner flexible bands 212, 214) and outer flexible band assembly 230 (including first and second flexible bands 232, 234) to pass therethrough (see FIG. 20). More particularly, and as shown in FIG. 57, each of first portion 1700a of seal assembly 1700 and second portion 1700b include openings 1722a, 1722b (e.g., C-shaped), respectively. When first portion 1700a and second portion 1700b engage each other, openings 1722a, 1722b form channels 1722 (FIG. 56). Accordingly, during assembly of surgical device 10, for instance, seal assembly 1700 is positionable to surround inner flexible band assembly 210 and outer flexible band assembly 230 without the need to thread inner flexible band assembly 210 and outer flexible band assembly 230 through channels 1722. Additionally, while four channels 1722 are shown, seal assembly 1700 may include more or fewer than four channels 1722 depending on the number of bands (or other features) extending therethrough.

Referring now to FIGS. 57 and 62, seal assembly 1700 also includes a plurality of channel seals 1724 associated with each channel 1722. In the illustrated embodiment, each channel 1722 includes three longitudinally-spaced channel seals 1724 extending along the periphery of channel 1722. Channel seals 1724 (e.g., rubber gaskets) are configured to provide a seal (e.g., a water-tight seal) between walls of seal assembly 1700 defining channels 1722 and bands 212, 214, 232, 234 extending therethrough. Seal assembly 1700 may include more or fewer than three channel seals 1724 per channel 1722.

With reference to FIGS. 56 and 57, seal assembly 1700 includes a plurality of aperture seals 1712 associated with aperture 1710. In the illustrated embodiment, aperture 1710 includes three longitudinally-spaced aperture seals 1712 extending along the periphery of aperture 1710. Aperture seals 1712 (e.g., rubber gaskets) are configured to provide a seal (e.g., a water-tight seal) between walls of seal assembly 1700 defining aperture 1710 and a component (or components) of surgical device 10 extending therethrough. Seal assembly 1700 may include more or fewer than three aperture seals 1712.

FIG. 57 also illustrates a plurality of portion seals 1750. A plurality of first portion seals 1750a is disposed on first portion 1700a of seal assembly 1700, and a plurality of second portion seals 1750b is disposed on second portion 1700b of seal assembly 1700. When first portion 1700a of seal assembly 1700 is engaged with second portion 1700b of seal assembly 1700, first portion seals 1750a engage or otherwise contact (e.g., compress) corresponding second portion seals 1750b, thereby creating a seal (e.g., a water-tight seal) therebetween. As shown, a first set of portion seals 1750 is disposed on a proximal part of first portion 1700a and second portion 1700b, and a second set of portion seals 1750 is disposed on a distal part of first portion 1700a and second portion 1700b.

The use of aperture seals 1712, channel seals 1724, and portion seals 1750 helps prevent contaminants from entering portions of surgical device 10 that are located proximal of seal assembly 1700.

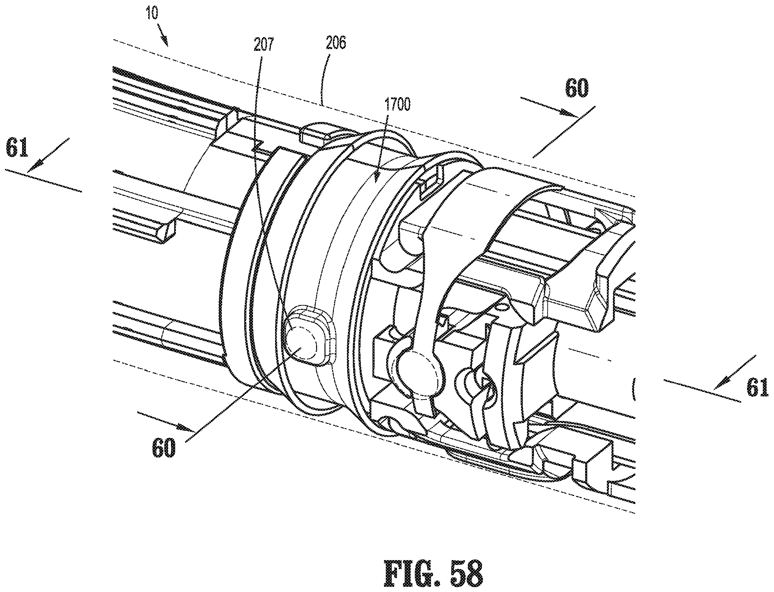

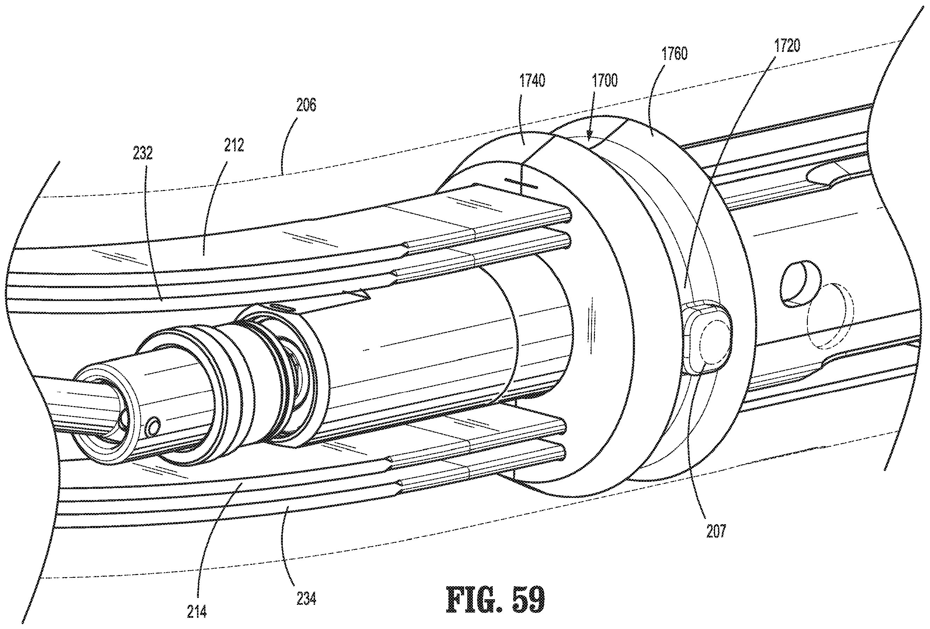

Seal assembly 1700 is positioned within outer sleeve 206 of surgical device 10 such that an opening or port 207 extending through outer sleeve 206 is positioned adjacent an annular space 1715 between proximal seal 1740 and distal seal 1760 of seal assembly 1700, as shown in FIGS. 58 and 59. Additionally, seal assembly 1700 is positioned such that each band 212, 214, 232, 234 of surgical device 10 extends through one channel 1722 of seal assembly 1700, as noted above.

To clean portions of surgical device (e.g., portions located distally of seal assembly 1700), a fluid (e.g., water, saline, etc.; or a gas) is introduced through port 207 of outer sleeve 206 into annular space 1715 of seal assembly 1700. With particular reference to FIG. 62, as fluid fills annular space 1715, proximal seal 1740 prevents the fluid from moving proximally therepast due to the angle α1 proximal seal 1740 makes with body portion 1720 of seal assembly 1700, and due to the interference (or contact) proximal seal 1740 makes with inner wall 206c of outer sleeve 206. Further, as the fluid pressure builds, the proximally-directed pressure causes proximal seal 1740 to be further forced against inner wall 206c of outer sleeve 206, thereby increasing the effectiveness of the seal.

With continued reference to FIG. 62, as fluid fills annular space 1715, the fluid pressure builds until distal seal 1760 is displaced away from inner wall 206c of outer sleeve 206 in the general direction of arrow "G." This displacement of distal seal 1760 away from inner wall 206c of outer sleeve 206 allows the pressurized fluid from annular space 1715 to sluice or flow between distal seal 1760 and inner wall 206c of outer sleeve 206, distally of seal assembly 1700, and through portions of extension assembly 200 and adapter assembly 100, for instance, thereby flushing these portions of surgical device 10 to remove surgical debris, for example. Since the fluid is introduced proximally of a distal end of seal assembly 1700, flow traps (which may otherwise occur between a fluid port and a seal disposed proximally thereof) are eliminated or minimized.

Methods of cleaning include inserting fluid through port 207 of outer sleeve 206 or an outer tube of surgical device 10 and into annular space 1715 between proximal seal 1740 and distal seal 1760, filling annular space 1715 with the fluid, deflecting distal seal 1760 away from its contact with outer sleeve 206 (in response to the pressure build-up of the fluid), and moving the fluid from annular space 1715 distally beyond distal seal 1760 of seal assembly 1700. The method also includes removing the fluid from a distal end of surgical device 10.

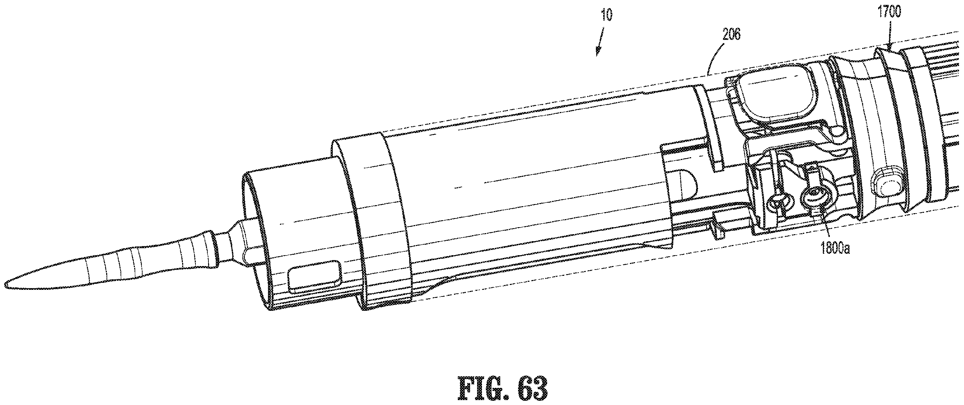

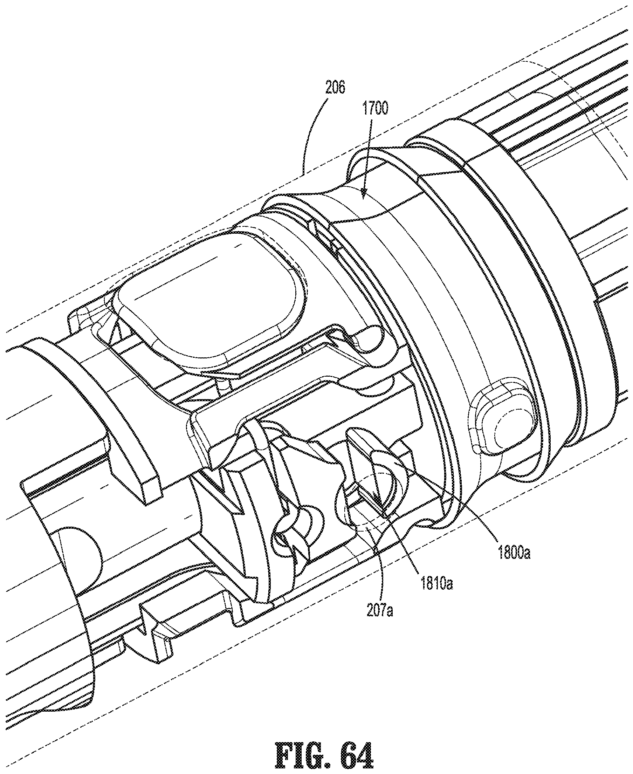

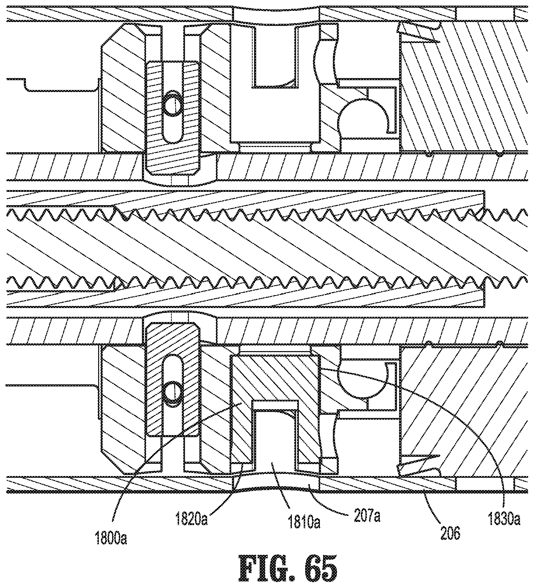

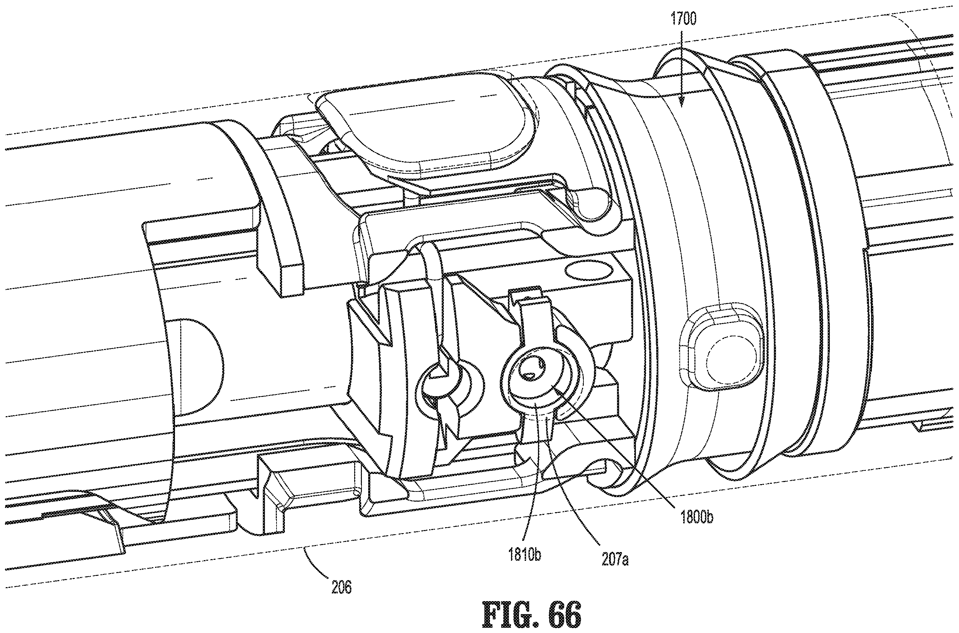

Referring now to FIGS. 63-68, direction nozzles 1800 for use with surgical device 10, adapter assembly 100, and/or extension assembly 200 are shown. A first type of direction nozzle 1800a is shown in FIGS. 63-65, and a second type of direction nozzle 1800b is shown in FIGS. 66-68. Collectively, first type of direction nozzle 1800a and second type of direction nozzle 1800b are referred to as direction nozzles 1800. Direction nozzles 1800 are configured to facilitate thoroughly cleaning debris (e.g., surgical debris) from surgical device 10 following use, prior to use, and/or prior to reuse, for instance. More specifically, direction nozzles 1800 are particularly useful when internal portions of surgical device 10 are flushed with a fluid to help remove debris from within surgical device 10. Further, direction nozzles 1800 are configured to minimize flow traps which may occur when attempting to flush a surgical device without a direction nozzle, for instance. Additionally, while direction nozzles 1800 are shown and described for use with a particular type of surgical device 10, direction nozzles 1800 are usable with various types of surgical instruments (e.g., reusable) where cleaning and/or sterilization may be desired.

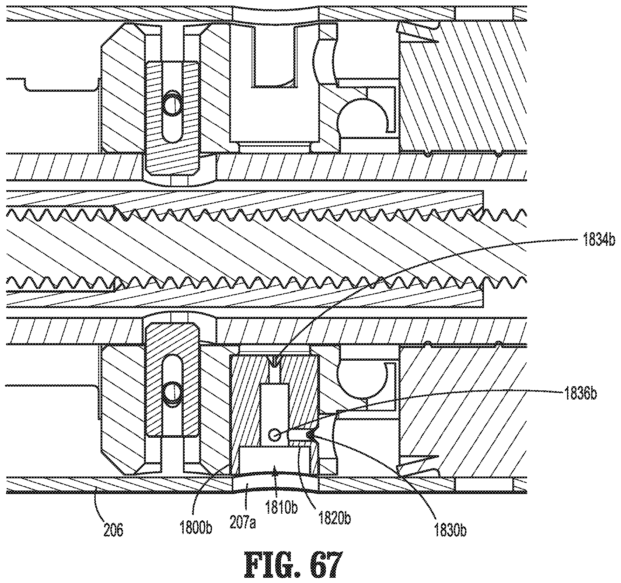

Direction nozzles 1800 are positioned within outer sleeve 206 of surgical device 10, distally of seal assembly 1700, and in a position where an opening or port 207a extending through outer sleeve 206 is positioned adjacent an input portion 1810 of one direction nozzle 1800. More particularly, in FIGS. 63-65, an input portion 1810a of direction nozzle 1800a is positioned adjacent port 207a of outer sleeve 206; in FIGS. 66 and 67, an input portion 1810b of direction nozzle 1800b is positioned adjacent port 207a of outer sleeve 206.

Outer sleeve 206 includes two ports 207a, for example, and one direction nozzle 1800 is positioned in fluid communication with each port 207a. Ports 207a are configured for engagement with a syringe or an irrigation pump, for example, to provide or introduce a fluid (e.g., water, saline, etc.; or a gas) therethrough. Direction nozzles 1800a, 1800b each include a shelf 1820a, 1820, respectively, configured to abut a portion of a syringe when the syringe is inserted through port 207a and through respective input portion 1810a, 1820b. Engagement between the syringe and shelf 1820a, 1820b prevents further insertion of the syringe and thereby protects various portions of the surgical device 10 and/or direction nozzles 1800a, 1800b from damage.

Direction nozzles 1800a, 1800b are configured to direct the fluid towards areas of surgical device 10 that are generally difficult to access (e.g., flow traps) using traditional flushing techniques. For example, first direction nozzle 1800a includes a single fluid outlet 1830a configured to direct all the fluid in a single direction (e.g., proximally).

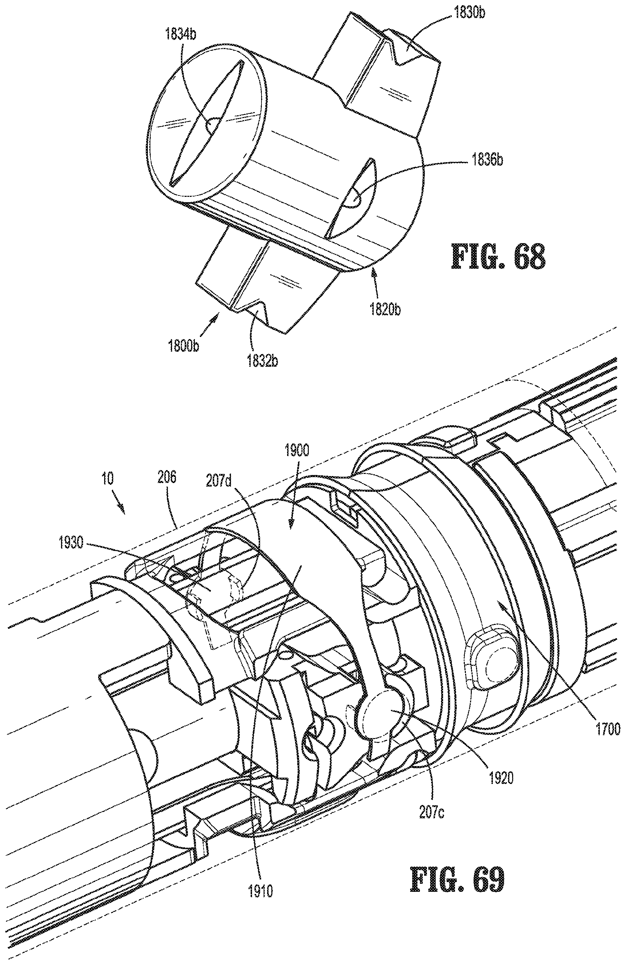

With particular reference to FIGS. 67 and 68, second direction nozzle 1800b includes a first fluid outlet 1830b, a second fluid outlet 1832b, a third fluid outlet 1834b, and a fourth fluid outlet 1836b, which each direct the fluid in particular directions. More specifically, first fluid outlet 1830b directs fluid proximally, second fluid outlet 1832b directs fluid distally, third fluid outlet 1834b directs fluid inward, and fourth fluid outlet 1836b directs fluid in a first circumferential direction (i.e., toward the viewer in FIG. 67). It is also envisioned that direction nozzle 1800b includes a fifth fluid outlet that directs fluid in a second circumferential direction (i.e., into the page in FIG. 67). More or fewer fluid outlets are also contemplated to target specific potential flow traps, for example.

Methods of cleaning a surgical instrument (e.g., surgical device 10) utilizing direction nozzles 1800 include inserting fluid through port 207a of outer sleeve 206 or an outer tube of surgical device 10 and into direction nozzles 1800, and directing the fluid to predefined locations within surgical device 10 based on the angulation of fluid outlets (e.g., 1830b, 1832b, 1834b, 1836b). The method also includes removing the fluid from a distal end of surgical device 10.

Additionally, while direction nozzles 1800 are shown and described for use with a particular type of surgical device 10, direction nozzles 1800 are usable with various types of surgical instruments (e.g., reusable) where cleaning and/or sterilization may be desired.

Referring now to FIG. 69, a plug 1900 for use with surgical device 10, adapter assembly 100, and/or extension assembly 200 of the present disclosure is shown. Plug 1900 is configured to allow a first opening or port 207c extending through outer sleeve 206 of extension assembly 200 to be used for cleaning or flushing surgical debris from surgical device 10, for example, while simultaneously blocking or occluding a second opening or port 207d extending through outer sleeve 206.

More specifically, plug 1900 is generally C-shaped and includes a body or backspan 1910 interconnecting a first occluding portion 1920 and a second occluding portion 1930. Plug 1900 is positioned within outer sleeve 206 of surgical device 10, distally of seal assembly 1700, and in a position where first port 207c and second port 207d of outer sleeve 206 are positioned in contact with first occluding portion 1920 and second occluding portion 1930, respectively.

More particularly, first occluding portion 1920 of plug 1900 is configured to releasably engage first port 207c of outer sleeve 206, and second occluding portion 1930 of plug 1900 is configured to releasably engage second port 207d of outer sleeve 206. Each of first occluding portion 1920 and second occluding portion 1930 is biased radially outward and into engagement with first port 207c and second port 207d, respectively, to provide a fluid-tight seal therewith. Moreover, each of first occluding portion 1920 and second occluding portion 1930 is moveable or deflectable radially inward away from respective first port 207c and second port 207d.

Plug 1900 is a leaf spring made of out sheet metal, for example. In embodiments, plug 1900 is a plunger with a coil spring, for instance.

In use, when plug 1900 is in place within outer sleeve 206 and when no extraneous forces are acting on plug 1900, first occluding portion 1920 is in mechanical engagement with and occluding (e.g., plugging or blocking) first port 207c of outer sleeve 206, and second occluding portion 1930 is in mechanical engagement with and occluding (e.g., plugging or blocking) second port 207d of outer sleeve 206. When cleaning debris from surgical device 10 is desired, a user can introduce fluid through first port 207c of surgical sleeve 206 using a syringe, for example. The engagement between the syringe (or the fluid exiting the syringe) and first occluding portion 1920 of plug 1900 moves first occluding portion 1920 radially inward and out of engagement with first port 207c, thereby allowing fluid to enter surgical device 10 through first port 207c. While fluid is entering surgical device 10 through first port 207c, second occluding portion 1930 maintains its engagement with second port 207d (e.g., due to its radially outward bias), thereby preventing the fluid from exiting surgical device 10 through second port 207d and such that the fluid exits surgical device 10 through the distal end, as intended.

Likewise, a user can introduce fluid through second portion 207d of surgical sleeve 206 using a syringe to deflect second occluding portion 1930 of plug radially inward, while first occluding portion 1920 maintains its engagement with first port 207c to prevent fluid from exiting surgical device through first port 207c.

Methods of cleaning a surgical instrument (e.g., surgical device 10) utilizing plug 1900 include inserting fluid through first port 207c of outer sleeve 206 or an outer tube of surgical device 10, moving first occluding portion 1920 of plug 1900 out of engagement with first port 207c while simultaneously maintaining engagement between second occluding portion 1930 of plug and second port 207d, introducing fluid within surgical device 10 through first port 207c, and removing the fluid from a distal end of surgical device 10.

Additionally, while plug 1900 is shown and described for use with a particular type of surgical device 10, plug 1900 is usable with various types of surgical instruments (e.g., reusable) where cleaning and/or sterilization may be desired.

Referring now to FIGS. 70-73, a luer 2000 for use with surgical device 10, adapter assembly 100, and/or extension assembly 200 of the present disclosure is shown. Luer 2000 is configured to facilitate thoroughly cleaning debris (e.g., surgical debris) from surgical device 10 following use, prior to use, and/or prior to reuse, for instance. More specifically, luer 2000 is particularly useful when internal portions of surgical device 10 are flushed with a fluid to help remove debris from within surgical device 10. Further, luer 2000 is configured to minimize flow traps which may occur when attempting to flush a surgical device without a luer, for instance. Additionally, while luer 2000 is shown and described for use with a particular type of surgical device 10, luer 2000 is usable with various types of surgical instruments (e.g., reusable) where cleaning and/or sterilization may be desired.

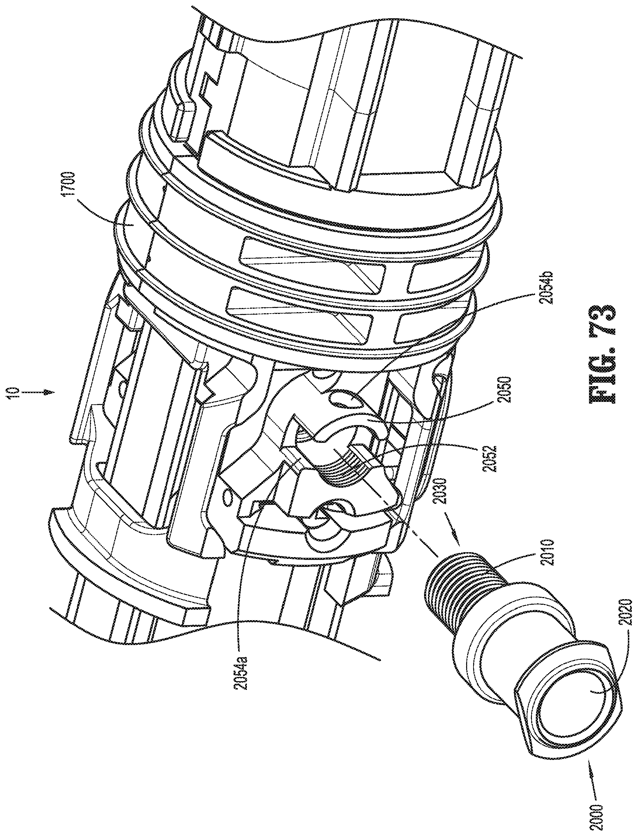

Luer 2000 is positioned such that a portion of luer 2000 is within an opening or port 207b extending through outer sleeve 206 of surgical device 10, and distally of seal assembly 1700. More particularly, a threaded portion 2010 of luer 2000 is positioned in operative engagement with a threaded or tapped portion 2052 of a housing 2050 (see FIG. 73) within outer sleeve 206. The threaded connection between threaded portion 2010 of luer 2000 and tapped portion 2052 of housing 2050 allows luer 2000 to be inserted and removed with relative ease when cleaning and/or sterilization is desired.

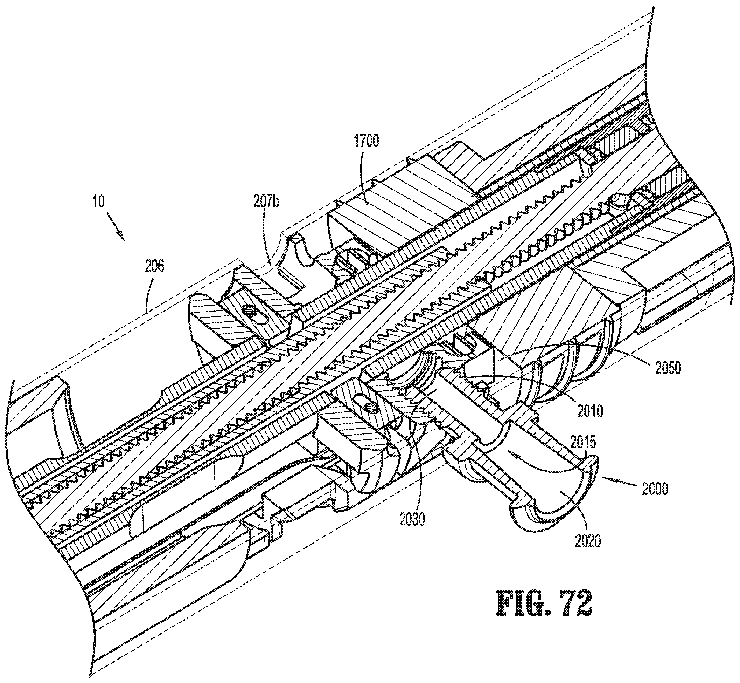

Luer 2000 also defines a channel 2015 (FIG. 72) including an input portion 2020, and an exit port 2030. Exit port 2030 is disposed adjacent threaded portion 2010 of luer 2000, and is generally disposed on the opposite side of luer 2000 as input portion 2020.

Outer sleeve 206 includes two ports 207b, for example, and one luer 2000 is positionable in fluid communication with each port 207b. Input portion 2020 of luer 2000 is configured for engagement with a syringe or an irrigation pump, for example, to provide or introduce a fluid (e.g., water, saline, etc.; or a gas) therethrough.

Exit port 2030 and housing 2050 are configured to direct the fluid within luer 2000 towards areas of surgical device 10 that are generally difficult to access (e.g., flow traps) using traditional flushing techniques. For example, housing 2050 includes a first aperture 2054a and a second aperture 2054b (see FIG. 73) defined by walls of housing 2050, which are configured to direct fluid which exits exit port 2030 in particular directions. For example, first aperture 2054a is configured to direct fluid radially, and second aperture 2054b is configured to direct fluid proximally.

It is further envisioned that surgical device 10 includes at least one threaded plug, for example, which can each be threaded into one ports 207b of outer sleeve 206 to occlude the opening defined by the ports 207b when the surgical device 10 is being used to perform a surgical procedure, for instance.

The present disclosure also includes methods of cleaning a surgical instrument (e.g., surgical device 10) utilizing luer 2000. For instance, disclosed methods include engaging luer 2000 with housing 2050 of surgical device 10, inserting fluid through input portion 2020 of luer 2000 and through exit port 2030 of luer 2000, and directing the fluid to predefined locations within surgical device 10 based on the positions of first aperture 2054a and second aperture 2054b of housing 2050, for example. The method also includes removing the fluid from a distal end of surgical device 10.

Additionally, while luer 2000 is shown and described for use with a particular type of surgical device 10, luer 2000 is usable with various types of surgical instruments (e.g., reusable) where cleaning and/or sterilization may be desired.

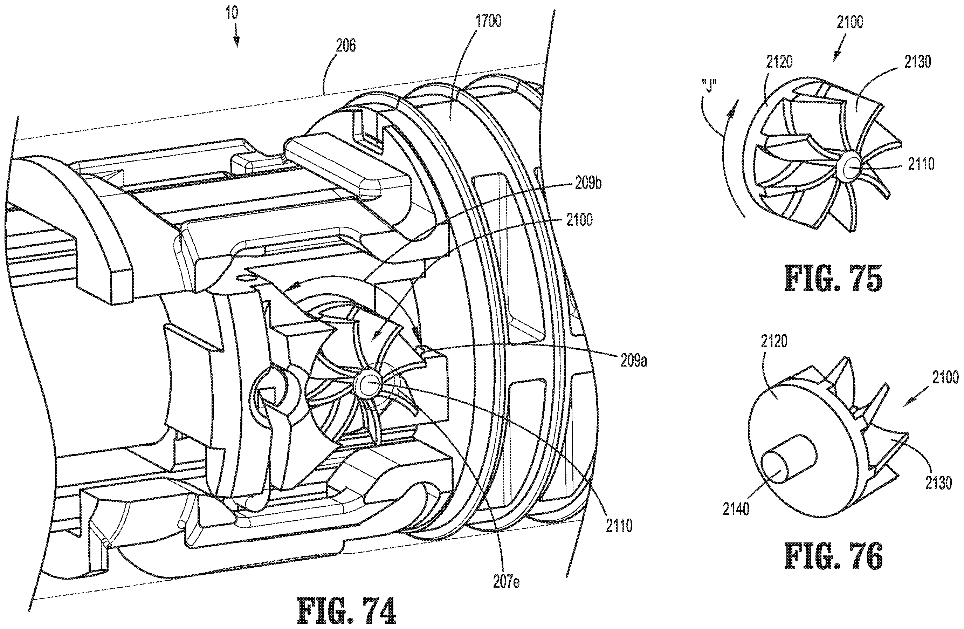

Referring now to FIGS. 74-77, an impeller 2100 for use with surgical device 10, adapter assembly 100, and/or extension assembly 200 is shown. Impeller 2100 is configured to facilitate thoroughly cleaning debris (e.g., surgical debris) from surgical device 10 following use, prior to use, and/or prior to reuse, for instance. More specifically, impeller 2100 is particularly useful when internal portions of surgical device 10 are flushed with a fluid to help remove debris from within surgical device 10. Further, impeller 2100 is configured to minimize flow traps which may occur when attempting to flush a surgical device without an impeller, for instance. Additionally, while impeller 2100 is shown and described for use with a particular type of surgical device 10, impeller 2100 is usable with various types of surgical instruments (e.g., reusable) where cleaning and/or sterilization may be desired.

Impeller 2100 is positioned within outer sleeve 206 of surgical device 10, distally of seal assembly 1700, and in a position where an opening or port 207e extending through outer sleeve 206 is positioned adjacent a central portion 2110 of impeller 2100. Outer sleeve 206 includes two ports 207e, for example, and one impeller 2100 is positioned in fluid communication with each port 207e. Ports 207e are configured for engagement with a syringe or an irrigation pump, for example, to provide or introduce a fluid (e.g., water, saline, etc.; or a gas) therethrough and into contact with impeller 2100.

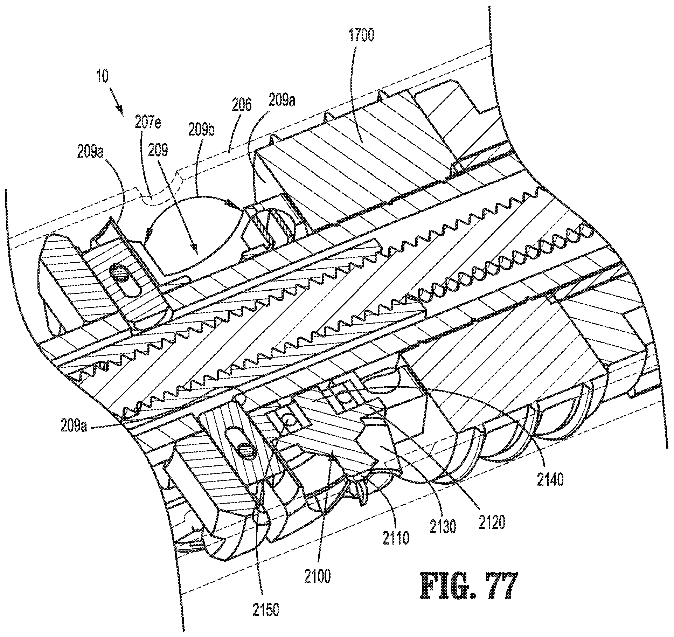

Impeller 2100 includes central portion 2110, a base 2120, a plurality of blades 2130 extending in a first direction from base 2120, and a pin 2140 defining a pin axis and extending in a second, opposite direction from base 2120. Additionally, a bearing 2150 (e.g., a ball bearing) is disposed between pin 2140 and walls 209a defining an aperture 209 within outer sleeve 206 (see FIG. 77). The engagement between pin 2140 and bearing 2150 helps ensure blades 2130 of impeller 2100 efficiently rotate about the pin axis during use. While the illustrated embodiment of impeller 2100 includes eight blades 2130, impeller 2100 of the present disclosure may include more or fewer than eight blades 2130.

The introduction of fluid within port 207e (e.g., by a syringe) causes impeller 2100 to rotation about pin 2140 in the general direction of arrow "J" in FIG. 75 (clockwise). That is, when the fluid contacts plurality of blades 2130 of impeller 2100, the curvature of plurality of blades 2130 causes the rotation to occur. While the plurality of blades 2130 in the illustrated embodiment is curved in a particular direction, the present disclosure also encompasses a plurality of blades 2130 curved in the opposite direction, which would result in rotation in a counter-clockwise direction. As fluid enters port 207e, and as the plurality of blades 2130 rotates, the fluid is directed through a gap 209b between walls 209a of aperture 209 (FIGS. 74 and 77). The location and size of gap 209b between walls 209a of aperture 209 defines the area where the fluid is directed. In the illustrated embodiment, gap 209b between walls 209a of aperture 209 directs at least some fluid proximally. Further, the rotation of the plurality of blades 2130 increases the velocity at which the fluid is directed within outer sleeve 206.

Methods of cleaning a surgical instrument (e.g., surgical device 10) utilizing impeller 2100 include engaging impeller 2100 with port 207e of outer sleeve 206 of surgical device 10, inserting fluid through port 207e and into contact with the plurality of blades 2130 of impeller 2100 causing the plurality of blades 2130 to rotate about the pin axis, and directing the fluid to predefined locations within surgical device 10 based on the size and location of gap 209b between walls 209a of aperture 209. The method also includes removing the fluid from a distal end of surgical device 10.

Additionally, while impeller 2100 is shown and described for use with a particular type of surgical device 10, impeller 2100 is usable with various types of surgical instruments (e.g., reusable) where cleaning and/or sterilization may be desired.

Surgical devices such as those described herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

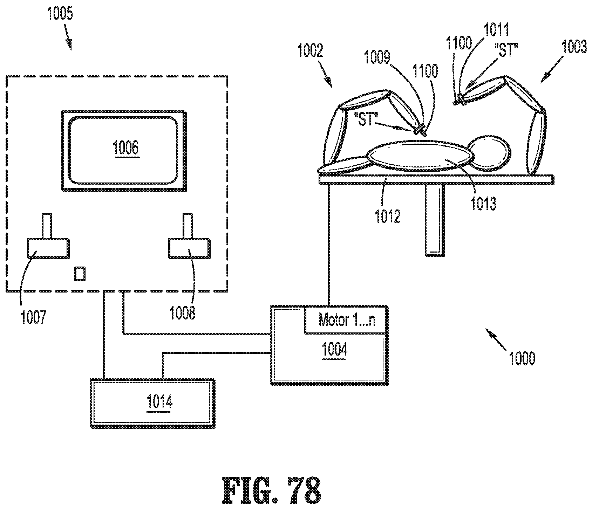

Referring to FIG. 78, a medical work station is shown generally as work station 1000 and generally may include a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100, in accordance with any one of several embodiments disclosed herein, as will be described in greater detail below.

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool (including end effector 1100) execute a desired movement according to a movement defined by means of manual input devices 1007, 1008. Control device 1004 may also be set up in such a way that it regulates the movement of robot arms 1002, 1003 and/or of the drives.

Medical work station 1000 may be configured for use on a patient 1013 lying on a patient table 1012 to be treated in a minimally invasive manner by means of end effector 1100. Medical work station 1000 may also include more than two robot arms 1002, 1003, the additional robot arms likewise being connected to control device 1004 and being telemanipulatable by means of operating console 1005. A medical instrument or surgical tool (including an end effector 1100) may also be attached to the additional robot arm. Medical work station 1000 may include a database 1014, in particular coupled to with control device 1004, in which are stored, for example, pre-operative data from patient/living being 1013 and/or anatomical atlases.

Reference is made herein to U.S. Patent No. 8,828,023 to Neff et al., entitled "Medical Workstation.

Any of the components described herein may be fabricated from either metals, plastics, resins, composites or the like taking into consideration strength, durability, wearability, weight, resistance to corrosion, ease of manufacturing, cost of manufacturing, and the like.

## Claims

1. A surgical device (10) comprising:
an outer sleeve (206) including an inner wall (206c) , a port and a housing within the port, the port extending through the inner wall of the outer sleeve; and
a luer (2000) configured for selective engagement with the housing, the luer including an input portion (2020) and an exit port (2023), the input portion is disposed radially outward of the outer sleeve when the luer is engaged with the housing and is configured for engagement with a source of fluid, the exit port is disposed radially inward of the inner wall of the outer sleeve (206) when the luer is engaged with the housing **characterized in that** the housing defines an aperture (2054a) configured to direct fluid proximally.

2. The surgical device according to claim 1, wherein the luer includes a threaded portion (2010) configured to selectively engage a tapped portion of the housing.

3. The surgical device according to any preceding claim, further comprising an end effector (30) disposed distally of the outer sleeve and configured to treat tissue.

4. The surgical device according to any preceding claim, further comprising a seal (1700) within the outer sleeve and disposed proximally of the end effector.

5. The surgical device according to claim 4, wherein the port is disposed distally of the seal.

## Patentansprüche

1. Chirurgische Vorrichtung (10), umfassend:
eine äußere Hülse (206), die einen Innenwand (206c), einen Anschluss und ein Gehäuse in dem Anschluss aufweist, wobei sich der Anschluss durch die Innenwnd der äußeren Hülse erstreckt, und
einen Luer (2000), der zum gezielten Eingriff mit dem Gehäuse ausgestaltet ist, wobei der Luer einen Eingangsabschnitt (2020) und einen Austrittsanschluss (2023) aufweist, wobei der Eingangsabschnitt radial außerhalb der äußeren Hülse angeordnet ist, wenn der Luer mit dem Gehäuse in Eingriff steht, und zum Eingriff mit einer Fluidquelle ausgestaltet ist, und der Austrittsanschluss radial innerhalb der Innenwand der äußeren Hülse (206) angeordnet ist, wenn der Luer mit dem Gehäuse in Eingriff steht, **dadurch gekennzeichnet, dass** das Gehäuse eine Öffnung (2054) definiert, die dazu ausgestaltet ist, Fluid proximal zu lenken.

2. Chirurgische Vorrichtung nach Anspruch 1, wobei der Luer einen Gewindeabschnitt (2010) aufweist, der zur gezielten Ineingriffnahme eines Einschraubabschnitts des Gehäuses ausgestaltet ist.

3. Chirurgische Vorrichtung nach einem der vorhergehenden Ansprüche, ferner umfassend einen Endeffektor (30), der distal von der äußeren Hülse angeordnet und zur Behandlung von Gewebe ausgestaltet ist.

4. Chirurgische Vorrichtung nach einem der vorhergehenden Ansprüche, ferner umfassend eine proximal zu dem Endeffektor angeordnete Dichtung (1700) in der äußeren Hülse.

5. Chirurgische Vorrichtung nach Anspruch 4, wobei der Anschluss distal von der Dichtung angeordnet ist.

## Revendications

1. Dispositif chirurgical (10) comprenant :
un manchon externe (206) comportant une paroi interne (206c), un orifice et un logement à l'intérieur de l'orifice, l'orifice s'étendant à travers la paroi interne du manchon externe ; et
un raccord Luer (2000) conçu pour une mise en prise sélective avec le logement, le raccord Luer comportant une partie d'entrée (2020) et un orifice de sortie (2023), la partie d'entrée étant disposée radialement vers l'extérieur du manchon externe lorsque le raccord Luer est mis en prise avec le logement et étant conçue pour une mise en prise avec une source de fluide, l'orifice de sortie étant disposé radialement vers l'intérieur de la paroi interne du manchon externe (206) lorsque le raccord Luer est mis en prise avec le logement, **caractérisé en ce que** le logement définit une ouverture (2054a) conçue pour diriger le fluide proximalement.

2. Dispositif chirurgical selon la revendication 1, le raccord Luer comportant une partie filetée (2010) conçue pour entrer en prise de façon sélective avec une partie taraudée du logement.

3. Dispositif chirurgical selon l'une quelconque des revendications précédentes, comprenant en outre un effecteur terminal (30) disposé distalement par rapport au manchon externe et conçu pour traiter un tissu.

4. Dispositif chirurgical selon l'une quelconque des revendications précédentes, comprenant en outre un joint d'étanchéité (1700) à l'intérieur du manchon externe et disposé proximalement par rapport à l'effecteur terminal.

5. Dispositif chirurgical selon la revendication 4, l'orifice étant disposé distalement par rapport au joint d'étanchéité.
